(19) Europäisches Patentamt
European Patent Office
Office européen des brevets

(11) Numéro de publication: **0 100 718**
**B1**

# (12) FASCICULE DE BREVET EUROPÉEN

(45) Date de publication du fascicule du brevet: **14.10.87**

(21) Numéro de dépôt: **83401520.8**

(22) Date de dépôt: **25.07.83**

(51) Int. Cl.⁴: **C 07 F 9/38**, C 07 F 9/40, C 07 F 9/65, C 07 F 9/58, A 61 K 31/66

## (54) Produits anti-inflammatoires dérivés de l'acide méthylène-diphosphonique et leur procédé de préparation.

(30) Priorité: **29.07.82 FR 8213250**

(43) Date de publication de la demande: **15.02.84 Bulletin 84/7**

(45) Mention de la délivrance du brevet: **14.10.87 Bulletin 87/42**

(84) Etats contractants désignés: **AT BE CH DE FR GB IT LI LU NL SE**

(56) Documents cité:
**EP-A-0 015 370**
**EP-A-0 054 997**
**EP-A-0 085 321**
**US-A-4 071 584**

**CHEMICAL ABSTRACTS, vol. 93, no. 25, 22 décembre 1980, page 877, no. 239648z, Columbus, Ohio, USA**
**SYNTHESIS, no. 2, février 1980, pages 127-129, Georg Thieme Verlag, Stuttgart, DE. M. MIKOLAJCZYK et al.: "Sulphenylation of phosphonates. A facile synthesis of alpha-phosphoryl sulphides and S,S-acetals of oxomethanephosphonates"**

**Le dossier contient des informations techniques présentées postérieurement au dépôt de la demande et ne figurant pas dans le présent fascicule.**

(73) Titulaire: **SANOFI, société anonyme, 40, Avenue George V, F-75008 Paris (FR)**

(72) Inventeur: **Breliere, Jean- Claude, 1065 rue de l'Aiguelongue Villa 12, F-34100 Montpellier (FR)**
Inventeur: **Emonds- Alt, Xavier, Le Boulidou- ap.11- Bat.4, F-34980 St Clement la Rivière (FR)**
Inventeur: **Garcia, George, 27 rue des Aires, F-34980 Saint Gely Du Fesc (FR)**

(74) Mandataire: **Gillard, Marie- Louise, Cabinet Beau de Loménie 55, Rue d'Amsterdam, F-75008 Paris (FR)**

(56) Documents cité: (suite)

**EP 0 100 718 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention a pour objet de nouveaux dérivés del'acide méthylènediphosphonique doués de propriétés thérapeutiques permettant leur utilisation dans le traitement des manifestations de l'inflammation.

Plus précisément, l'invention concerne pour leur utilisation comme agents anti-inflammatoires, les composés de formule générale:

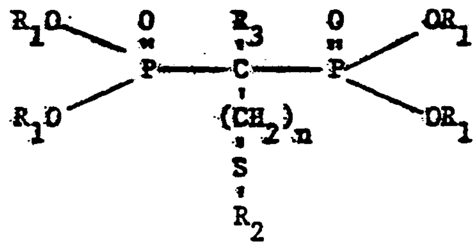

(I)

dans laquelle

. $R_1$ représente l'hydrogène ou un groupe alkyle inférieur droit ou ramifié ayant de 1 à 4 atomes de carbone;

. $R_2$ représente l'hydrogène;

un groupe alkyle éventeullement substitué par un groupe hydroxyle, thiol, un ou plusieurs atomes d'halogène, un groupe alcoxycarbonyle ou un groupe

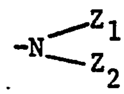

ou $Z_1$ et $Z_2$ considérés indépendamment représentent l'hydrogène ou un groupe alkyle inférieur;

un groupe phényle éventuellement substitué une ou plusieurs fois par un halogène, un groupe nitro, un groupe alkyle inférieur, alcoxy inférieur, trifluorométhyle ou encore par un groupe $NH_2$ ou un groupe COOH ou COO alkyle;

un groupe

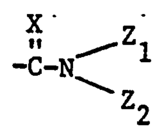

où X designe l'oxygène ou le soufre et $Z_1$ et $Z_2$ ont été définis ci-dessus;

un hétérocycle à 5 ou 6 chaînons comportant 1 ou 2 hétéroatomes choisis parmi l'azote et le soufre;

un hétérocycle à 5 chaînons condensé avec un noyau benzénique et répondant à la formule

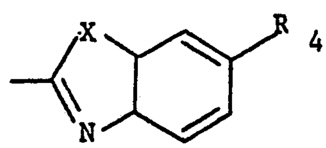

où X peut représenter l'oxygène, un groupe NH ou le soufre et $R_4$ désigne l'hydrogène ou un atome d'halogène, de préférence le chlore;

. $R_3$ désigne l'hydrogène ou un groupe hydroxyle;

. enfin, n représente un nombre entier compris entre 0 et 10, à la condition que n ne peut pas être égal à 0 lorsque $R_3$ représente OH. L'invention a également pour objet les composés de formule I ci-dessus dans laquelle:

1) $R_1$ est l'hydrogène ou le groupe isopropyle,

$R_2$ est le groupe 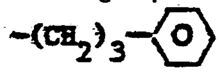

$R_3$ est l'hydrogène et n = 0 ou

2) $R_1$ et $R_3$ sont l'hydrogène, $R_2$ est le méthyl-1, imidazolyle et n = 6.

Lorsque $R_1$ représente l'hydrogène, les acides de formule (I) sont susceptibles de fournir des sels avec les bases minérales ou organiques. Ces sels font partie intégrante de l'invention.

Quelques une des composés de formule (I) ont déjà été décrits, mais en aucun cas leurs propriétés thérapeutiques n'ont été mentionnées.

Ainsi le brevet US N° 407.1584 décrit, sans les revendiquer, les composés de formule (I) ou $R_1$ est $CH_3$ ou $C_2H_5$, $R_3$ est H et $R_2$ désigne le groupe

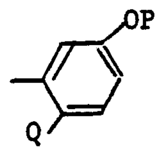

dans lequel P = $CH_3$ et Q = Br, $NO_2$ ou encore P = H et Q: Br, $NO_2$, Cl et n = 0.

Ce brevet décrit également l'acide (I): $R_1$ = H, P = H, Q = Cl. Ces produits sont des produits de transformation de dérivés phosphorés eux-mêmes revendiqués comme inhibiteurs de corrosion, additifs pour huile ou "flame retardants".

Dans la revue Synthesis de Février 1980, pages 127-129, les auteurs décrivent entre autres le composé (I) où $R_1$ = $C_2H_5$, $R_3$ = H, $R_2$ = $C_6H_5$, n = 0, sans en indiquer aucune propriété thérapeutique.

La demande de brevet japonais 80 98193 [Chemical Abstracts <u>93</u>, 23648z, (1980)] cite en tant qu'herbicide les composés (I) où $R_1$ = H, $R_2$ = pyridyle, $R_3$ = H et n = 0.

Le Journal Für Praktische Chemie, 1970 (3), 475-482, décrit le composé (I) où $R_1$ = H, $R_2$ = $CH_3$, $R_3$ = H, n = 0. Il s'agit d'un article purement chimique.

Les autres composés (I) sont nouveaux et constituent un autre objet de la présente invention.

La présente invention comporte également un procédé de préparation des composés de formule (I). Lorsque $R_3$ = H, on utilise le procédé suivant:

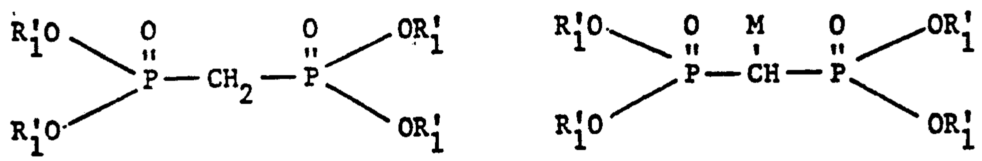

<u>1</u>

$R'_1$ = alkyle inférieur

M = Na ou K

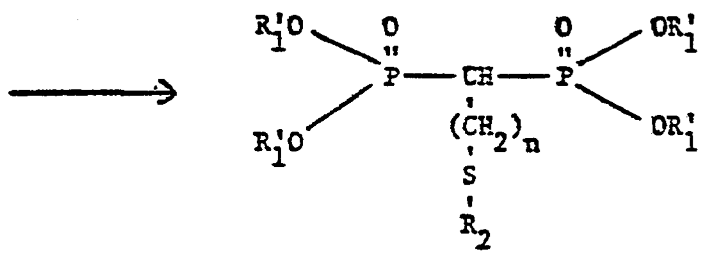

(I)

$R_1$ = alkyle inférieur

On utilise comme produit de départ un ester d'alkyle inférieur de l'acide méthylènediphosphonique <u>1</u> dont on prépare le dérivé sodé correspondant par action d'un agent de sodation tel que l'hydrure de sodium, au sein d'un solvant convenable, tel qu'un hydrocarbure benzénique, de préférence le toluène, ou encore dans le diméthylformamide.

La réaction est effectuée à une température comprise entre 0 et 50°C et, de préférence à température ambiante (20°C). On peut aussi préparer le dérivé métallique par action de la potasse au sein du toluène.

On effectue ensuite la substitution par le groupe $-(CH_2)_n-S-R_2$ à partir du dérivé métallique obtenu ci-dessus et non isolé.

Lorsque n = 0 on fait réagir sur le dérivé sodé le disulfure $R_2S-S-R_2$ au sein du solvant utilisé pour préparer le dérivé sodé. La température et la durée de réaction varient notablement suivant les réactifs utilisés. La température de réaction est située entre 20°C et la température d'ébullition du solvant tandis que la durée de réaction varie de quelques heures à plusieurs jours.

On peut également remplacer le disulfure par le chlorure de sulfényle correspondant $R_2SCl$.

Lorsque n est autre que 0, on fait agir sur le dérivé sodé un composé halogéné $Hal(CH_2)_n-S-R_2$, à une température comprise entre la température ambiante et celle de reflux du solvant. Une variante de ce procédé consiste à utiliser un dérivé dihalogéné $Hal-(CH_2)_n-Hal$ puis à faire réagir le thiol $R_2SH$ sur le composé intermédiaire ainsi obtenu.

Enfin, lorsque n = 3 une variante du procédé consiste à substituer l'ester diphosphonique comme indiqué par un halogénure d'allyle, puis à faire réagir ensuite le thiol $R_2SH$ sur l'allyle diphosphonate ainsi obtenu. Lorsque le thiol $R_2SH$ utilisé est volatil, on peut être amené à opérer dans un autoclave à une température comprise entre 80 et 150°C.

Dans tous les cas lorsque le groupement $-(CH_2)_n-S-R_2$ introduit comporte des substituants susceptibles de réagir ils doivent être préalablement bloqués par un réactif qui peut être ensuite facilement éliminé.

Ainsi les groupes OH peuvent être bloqués par exemple par formation d'un éther du dihydropyranne et les

groupes carboxyles peuvent être bloqués sous forme de sel de sodium.

Quand $R_3$ est OH on prépare les composés (I) selon le schéma réactionnel:

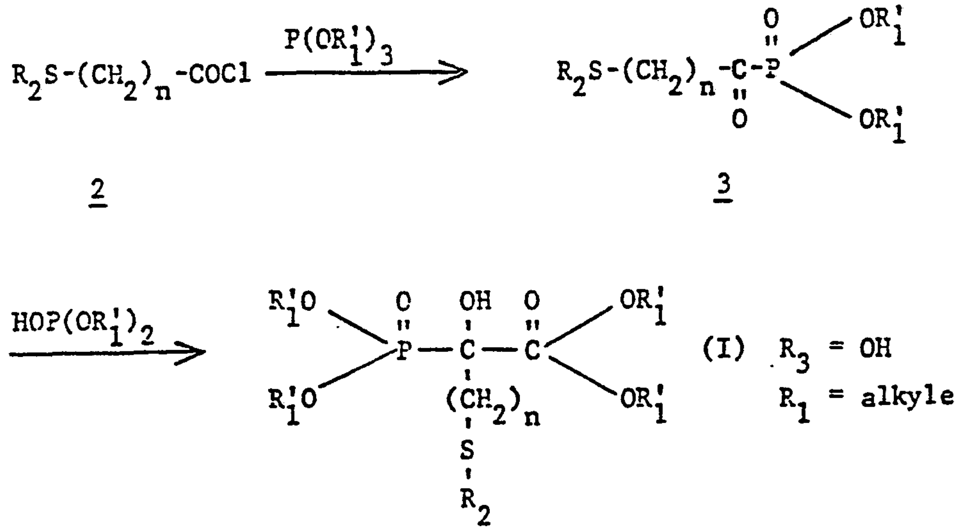


2

3

(I) $R_3$ = OH
$R_1$ = alkyle

On fait agir le chlorure d'acide 2 sur un trialkylphosphite à une température comprise entre 20 et 50°C. On obtient ainsi le composé 3. Celui-ci non isolé est traité par un dialkylphosphite en présence d'une amine secondaire telle que la dibutylamine à une température comprise entre 0 et 20°C.

Une variante de ce procédé consiste à remplacer le composé 2 par un chlorure d'acide halogéné Hal-$(CH_2)_n$-COCl (4) qui conduit en deux étapes de la même façon au composé:

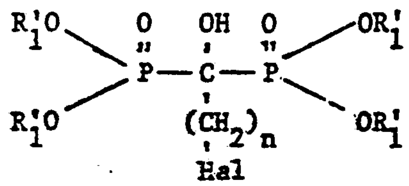

Celui-ci traité par un thiol $R_2SH$ dans un solvant tel que l'acétonitrile et en présence de diaza-1,5 bicyclo(5,4,0)undécène-5 et à basse température conduit aux composés (I): $R_3$ = OH $R_1$ = alkyle.

A partir des composés (I) où $R_1$ est alkyle on passe aux composés (I) où $R_1$ représente l'hydrogène par saponification. On opère par chauffage au reflux de l'ester dans l'acide chlorhydrique dilué pendant une période variant suivant les cas de quelques heures à 24 h. Après isolement par évaporation l'acide ainsi obtenu peut être transformé de façon connue en l'un de ses sels. On opère dans un solvant chaud de façon que le sel cristallise par refroidissement.

La saponification des esters peut également s'effectuer par action du bromure de triméthylsilyle au sein d'un solvant tel que le tétrachlorure de carbone à une température peu élevée le plus souvent à température ambiante.

Les exemples suivants sont donnés à titre d'illustration de l'invention.

## EXEMPLE 1

Benzoxazolyl-2 thiométhylènediphosphonate de tétraméthyle.
(SR 41625)

(I) $R_1 = CH_3$; n = 0; $R_2$ = 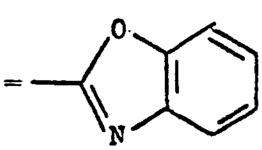

Dans un mélange de 24 g de méthylènediphosphonate de tétraméthyle et 50 ml de diméthylformamide on ajoute sous atmosphère d'azote et à 15° C 2,5 g d'hydrure de sodium. On laisse sous agitation pendant 1 h à 15° C puis on ajoute à 20° C 44 g de dibenzoxazolyl-2 disulfure. On poursuit l'agitation à 25° C pendant 68 h puis on évapore le diméthylformamide à 20° C sous vide. Le résidu dissous dans le chlorure de méthylène est chromatographié sur une colonne de 500 g de silice.

En éluant avec du chlorure de méthylène on élimine les produits de départ n'ayant pas réagi puis en éluant avec un mélange chlorure de méthylène-éthanol 96-4 (vol/vol) on obtient le produit attendu encore impur.

On purifie en effectuant une nouvelle chromatographie sur 160 g de silice. Avec le mélange solvant chlorure de méthylène-éthanol 97-3 (vol/vol) on obtient un produit qui cristallise en refroidissant la solution à 0° C; F: 76-8° C.

En opérant de la même façon avec l'ester tétraisopropylique de l'acide méthylènediphosphonique et en faisant varier le disulfure utilisé on obtient les différents esters réunis dans le tableau 1.

**TABLEAU 1**

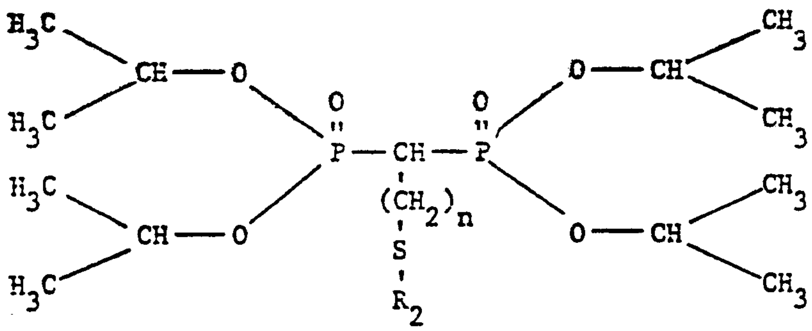

| n° de code | n | $R_2$ | Conditions opératoires (température et temps de chauffage) | Caractéristiques physiques |
|---|---|---|---|---|
| SR-41265 | 0 | 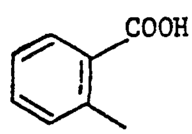 | 90°C-5 h | 140°C (acétonitrile) |
| SR-41452 | 0 | 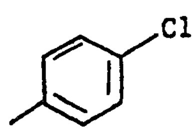 | 25°C-6 h | Huile jaune Chromatographie sur couche mince de silice ACOET-Et OH 8-2 (vol/vol) Rf = 0,7 |

**EXEMPLE 2**

Phénylthio-4 butylènediphosphonate-1,1 de tétra-isopropyle.
(SR 41341)

(I) $R_1$ = 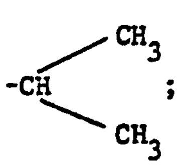 ; n = 3; $R_2$ = 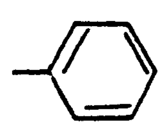

On prépare le dérivé sodé du méthylènediphosphonate de tétraisopropyle (34,4 g) comme dans l'exemple 1 puis on ajoute 28,7 g de bromo-1 phénylthio-3 propane et chauffe à 100° C pendant 1 h.

On évapore le solvant à siccité sous vide puis on reprend le résidu dans 500 ml d'eau et extrait avec du chlorure de méthylène. On sépare le phase organique sèche sur sulfate de sodium et évapore le solvant sous vide. Le résidu est chauffé à 130°C sous vide poussé pour éliminer les produits volatils puis on chromatographie sur colonne de gel de silice en éluant avec un mélange chloroforme-méthanol 99-1 (vol/vol).

On obtient ainsi une huile (23 g) qu'on purifie à nouveau par chromatographie sur gel de silice avec le même système solvant, par chromatographie sur couche mince de silice avec le système solvant butanone-2-eau 95-5 (vol/vol) on obtient une tache de Rf 0,57.

**EXEMPLE 3**

<u>n-Octylthiométhylènediphosphonate de tétraisopropyle.</u>
(SR 41454)

(I) $R_1$ = 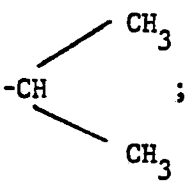 ; n = 0; $R_2$ = $-(CH_2)_7-CH_3$

On opère suivant la méthode de l'exemple 1 en utilisant comme solvant un mélange de toluène anhydre et de diméthylformamide.

Après chauffage pendant 16 h à 100°C on obtient par le même traitement le produit attendu sous forme d'huile caractérisée en chromatographie sur couche mince de silice dans le système solvant acétate d'éthyle-éthanol 8-2 (vol/vol) par un Rf de 0,65.

De même en remplaçant le dioctyldisulfure par une quantité équivalente de di-p-tolyldisulfure on obtient le p-tolylthiométhylènediphosphonate de tétraisopropyle (SR 41455) huile caractérisée en chromatographie sur couche mince de silice par un système solvant acétate d'éthyle-éthanol 8-2 (vol/vol) par un Rf de 0,57.

De même avec le di(trifluorométhyl-3 phényl)-disulfure on obtient le (trifiuorométhyl-3 phénylthio)méthylènediphosphonate de tétraisopropyle; Rf: 0,51 (acétate d'éthyle-éthanol 95-5 vol/vol) (SR 42248).

Enfin, en utilisant le di(trifluorométhyl-3 nitro-4 phényl)disulfure, on obtient de même le (trifluorométhyl-3 nitro-4 phénylthio)méthylènediphosphonate de tétraisopropyle; Rf: 0,51 (acétate d'éthyle-éthanol 95-5 vol/vol) (SR 42247).

**EXEMPLE 4**

<u>(Nitro-4 Phénylthio)-7 heptylidènediphosphonate-1,1 de tétraisopropyle.</u>
(SR 42147)

(I) $R_1$ = 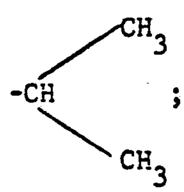 ; n = 6; $R_3$ = H; $R_2$ = 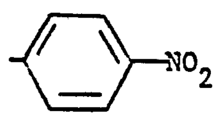

A la solution de 27 g de méthylènediphosphonate de tétraisopropyle dans 200 ml de toluène, on ajoute par fractions 3,8 g d'une suspension à 50 % d'hydrure de sodium dans l'huile. Après la fin de l'addition, on laisse 1 h sous agitation, puis on ajoute 27 g de (nitro-4 phénylthio)-1 bromo-6 hexane et chauffe pendant 3 h à 80° C.

On évapore à siccité sous vide et reprend le résidu dans l'hexane. On lave la solution deux fois avec de l'eau, puis sèche sur sulfate de sodium. On évapore le solvant à siccité et chromatographie le résidu sur une colonne de silice en éluant avec un mélange chlorure de méthylène-méthanol 98-2 (vol/vol).

On obtient ainsi 8,5 g d'une huile caractérisée en chromatographie sur couche mince de silice par un système solvant acétate d'éthyle-éthanol 95-5 vol/vol par un Rf de 0,37.

En opérant de la même façon mais en faisant varier le dérivé bromé utilisé on obtient:
- le (méthoxy-4 phénylthio)-7 heptylidènediphosphonate-1,1 de tétraisopropyle (SR 42306) Rf: 0,37 (acétate d'éthyle-éthanol 95-5 vol/vol);

- le (chloro-4 phénylthio)-7 heptylidènediphosphonate-1,1 de tétraisopropyle (SR 41964)
Rf: 0,37 (acétate d'éthyle-éthanol 95-5 vol/vol);
- le (dichloro-3 4-phénylthio)-7 heptylidènediphosphonate-1,1 de tétraisopropyle (SR 42146)
Rf: 0,37 (acétate d'éthyle-éthanol 95-5 vol/vol)
- le (chloro-4 phénylthio)-7 undécylidènediphosphonate-1,1 de tétraisopropyle (SR 42145)
Rf: 0,37 (acétate d'éthyle-éthanol 95-5 vol/vol).

**EXEMPLE 5**

(Phényl-3 propylthio)méthylènediphosphonate de tétraisopropyle
(SR 41907)

On agite pendant 20 h à 25°C le mélange de 29,2 g de méthylènediphosphonate de tétraisopropyle, 80 ml de toluène 11,2 g de potasse et 23,2 g de di(phényl-3 propyl)disulfure.

On lave le mélange cinq fois avec 50 ml d'eau, puis sèche la solution sur sulfate de sodium et évapore à siccité sous vide.

On obtient une huile chromatographiée sur une colonne de 500 g de silice. Après élimination d'impuretés éluées au chlorure de méthylène,on obtient par élution avec un mélange chlorure de méthylène-éthanol 95-5 (vol/vol) le produit attendu (11,2 g) chromatographié sur couche mince de silice: Rf = 0,5 (acétate d'éthyle-éthanol 95-5 vol/vol). De la même façon mais en utilisant le di(méthoxycarbonyl-2 éthyl)disulfure, on obtient le (méthoxycarbonyl-2 éthylthio)méthylènediphosphonate de tétraisopropyle (SR 42250); Rf : 0,37 (acétate d'éthyle-éthanol 95-5 vol/vol).

**EXEMPLE 6**

(N,N-diéthylthiocarbamoylthio)méthylènediphosphonate de tétraéthyle.
(SR 41905)

On opère comme dans l'exemple 3 à partir de méthylènediphosphonate de tétraéthyle et de bis(diéthylthiocarbamoyl)disulfure. De la même façon on obtient une huile de Rf: 0,5 (acétate d'éthyle-éthanol 8-2 vol/vol) en chromatographie en couche mince de silice.

**EXEMPLE 7**

Perfluorohexylthio)méthylènediphosphonate de tétraisopropyle.
(SR 42327)

On agite à 25°C sous atmosphère d'azote le mélange de 16 g de méthylènediphosphonate de tétraisopropyle et 1,05 g d'hydrure de sodium. On ajoute, après 1 h, 10 ml de chlorure de perfluorohexylsulfényle. La température s'élève spontanément à 80°C. On maintient 3 h à cette température puis on verse le mélange dans 50 ml d'eau et extrait avec de l'éther. On évapore le solvant et reprend le résidu dans 100 ml d'hexane. On lave la solution cinq fois avec 100 ml d'eau, puis sèche la solution et concentre à siccité sous vide.

On chromatographie le résidu sur une colonne de silice. On élimine les produits n'ayant pas réagi par le chlorure de méthylène, puis on élue le produit attendu par un mélange chlorure de méthylène-éthanol 97-3 vol/vol.

Le produit est obtenu sous forme d'huile.
Rf: 0,60 (acétate d'éthyle-éthanol 95-5 vol/vol).

**EXEMPLE 8**

Acide méthylthiométhylènediphosphonique trisel de tertiobutylamine.
(SR 41036)
(I) $R_1 = H$; $n = 0$; $R_2 = CH_3$

Dans un mélange de 8,8 g de méthylènediphosphonate de tétraisopropyle et 25 ml de toluène, on ajoute, à 0°C, 0,65 g d'hydrure de sodium puis on agite durant 1 h à 15°C. On ajoute 25 ml de diméthyldisulfure puis chauffe à 60°C durant 24 h.

On concentre à siccité sous vide puis on reprend le résidu dans 250 ml d'éther isopropylique. On filtre un insoluble et concentre à siccité. On chromatographie sur colonne d'alumine (150 g). On élue d'abord avec de l'éther isopropylique pour éliminer des impuretés puis avec du chlorure de méthylène fournissant le produit attendu.

On chauffe au reflux pendant 5 h 3 g de l'ester obtenu ci-dessus dans 12 ml de solution aqueuse d'acide chlorhydrique 6N. On lave la solution trois fois avec 30 ml de pentane, puis on décolore la phase aqueuse avec du charbon activé et concentre sous vide à siccité.

On salifie l'acide brut ainsi obtenu en y ajoutant 2,4 g de tertiobutylamine dans 200 ml d'éthanol absolu bouillant. Par refroidissement on obtient le trisel de tertiobutylamine sous forme d'un solide incolore; F.: 212°C.

De la même façon en faisant varier les disulfures utilisés on prépare les acides (I) réunis dans le tableau 2.

**TABLEAU 2**

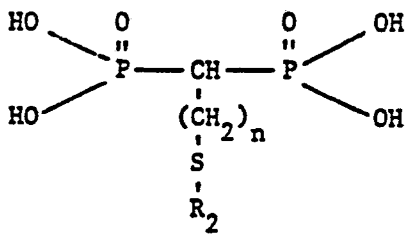

| n° de code | n | $R_2$ | Conditions opératoires: Substitution température et durée | Conditions opératoires: Hydrolyse conc. HCl et durée | Produit isolé |
|---|---|---|---|---|---|
| SR 41100 | 0 | 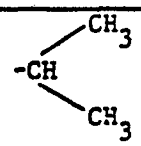 | 110°C<br>24 h | HCl 6N<br>5 h | disel de tertio-butylamine<br>F. : 252°C |
| SR 41179 | 0 | 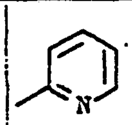 | 20°C<br>16 h | HCl 6N<br>24 h | chlorhydrate<br>F. > 280°C |
| SR 41264 | 0 | 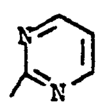 | 20°C<br>3 h | HCl 6N<br>8 h | chlorhydrate<br>F. > 300°C |
| SR 41266 | 0 | $-(CH_2)_2NH_2$ | 20°C<br>2 h | HCl 8N<br>18 h | chlorhydrate<br>F. : 285-290°C |
| SR 41344 | 0 | 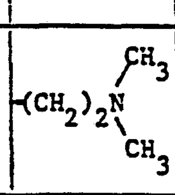 | 20°C<br>20 h | HCl 8N<br>18 h | chlorhydrate<br>F. 176°C |
| SR 41482 | 0 | 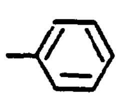 | 110°C<br>48 h | HCl 6N<br>6 h | trisel de tertio-butylamine<br>F. : 24°C (avec 1/2 $H_2O$) |
| SR 41688 | 0 | 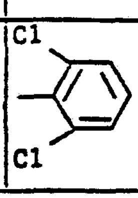 | 20°C<br>16 h | HCl 6N<br>16 h | acide libre<br>F. > 260°C |

TABLEAU 2 (suite)

| n° de code | n | $R_2$ | Conditions opératoires | | Produit isolé |
|---|---|---|---|---|---|
| | | | Substitution température et durée | Hydrolyse conc. HCl et durée | |
| SR 41689 | 0 | 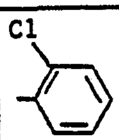 | 20°C<br>16 h | HCl 6N<br>16 h | acide libre<br>F. : 226°C |
| SR 41690 | 0 | 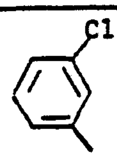 | 20°C | HCl 6N | disel de t-butylamine<br>F. : 270°C |

**EXEMPLE 9**

Acide (chloro-4 phényl)thiométhylènediphosphonique, disel de tertiobutylamine.
(SR 41319)

(I) $R_1$ = H; n = 0; $R_2$ = 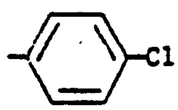

On opère comme dans l'exemple 4 en remplaçant comme solvant le toluène par le diméthylformamide.

Pour effectuer la substitution on chauffe avec le disulfure à 25° C pendant 6 h. On isole l'ester comme indiqué dans l'exemple 4 puis on saponifie avec HCl 12N pendant 18 h.

On obtient de la même façon l'acide qu'on transforme en disel de t-butylamine comme indiqué à l'exemple 4. F.: 253° C (décomposition).

De la même façon en faisant varier les disulfures utilisés on obtient les composés (I) selon l'invention réunis dans le tableau 3.

**TABLEAU 3**

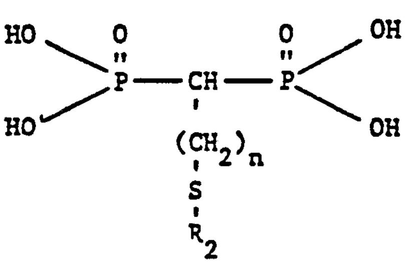

| n° de code | n | $R_2$ | Conditions opératoires | | Produit isolé |
|---|---|---|---|---|---|
| | | | Substitution température et durée | Hydrolyse conc. et durée | |
| SR 41263 | 0 | 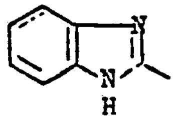 | 20°C<br>24 h | HCl 6N<br>12 h | Acide libre<br>F. > 300°C |
| SR 41388 | 0 | 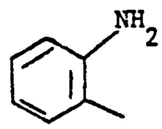 | 20°C<br>4 h | HCl 10N<br>12 h | Acide libre<br>F. : 272°C (déc.) (cristallise avec 1 molécule d'eau) |
| SR 41480 | 0 | 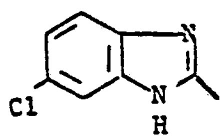 | 20°C<br>16 h | HCl 6N<br>16 h | Acide libre<br>F. > 260°C |
| SR 41552 | 0 | $CH_3-(CH_2)_6-$ | 20°C<br>18 h | HCl 12N<br>20 h | Sel de tertiobutylamine<br>F. : 218°C |

**EXEMPLE 10**

Acide méthylthio-3 propylidènediphosphonique-1,1 sel de tertiobutylamine.
(SR 41273)
(I) $R_1$, H; n = 2; $R_2 = CH_3$

On opère comme dans l'exemple 4 en remplaçant le disulfure par du bromo-1 méthylthio-2 éthane.

Après chauffage 20 h à 30°C, on isole l'ester, puis on l'hydrolyse comme indiqué dans l'exemple 4 par HCl 8N su reflux pendant 7 h.

Finalement on transforme l'acide en sel par action de la tertiobutylamine. F.: 212-4°C.

**EXEMPLE 11**

Acide phénylthio-4 butylidènediphosphonique-1,1 disel de tertiobutylamine.
(SR 41342)

(I) $R_1$ = H; n = 3; $R_2$ = 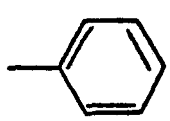

On chauffe au reflux, pendant 7 h, une solution de 5 g d'ester SR 41341 (exemple 1) dans 18 ml d'acide chlorhydrique 6N. On évapore à siccité et reprend le résidu dans 25 ml d'eau. On essore et sèche le précipité et recristallise dans l'acétonitrile puis on salifie par la tertiobutylamine; F.: 223° C.

De la même façon en faisant varier l'ester de départ on obtient les produits réunis dans le tableau 4.

**TABLEAU 4**

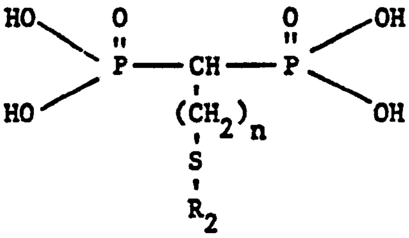

| n° de code | n | $R_2$ | Hydrolyse conc. HCl et durée | Produit isolé |
|---|---|---|---|---|
| SR 41421 | 0 | $-(CH_2)_7CH_3$ | HCl 12N 16 h | acide libre F. : 155-160°C |
| SR 41456 | 0 | 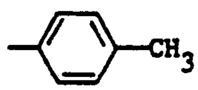 | HCl 12N 16 h | disel de t-butylamine F.: 255-260°C (déc.) |
| SR 41272 | 0 | 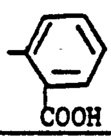 | HCl 6N 8 h | trisel de t-butylamine 198-202°C |
| SR 41908 | 0 | 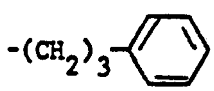 | HCl 12N 16 h | acide libre F. : 176-178°C |
| SR 41960 | 0 | 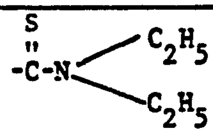 | HCl 12N 16 h | disel de t-butylamine F. : 235°C (déc.) |
| SR 42249 | 0 | 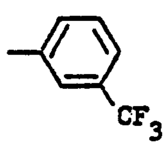 | HCl 12 N 10 h | acide libre F. : 202-204°C |
| SR 41959 | 6 | 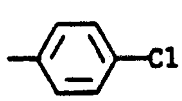 | HCl 6N 12 h | disel de t-butylamine F. : 220-224°C |
| SR 42143 | 6 | 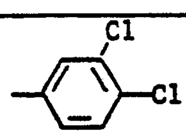 | HCl 12 N 12 h | disel de t-butylamine F. : 224-226°C |

**EXEMPLE 12**

Acide hexadécylthiométhylènediphosphonique monosel d'ammonium.
(SR 41453)
(I) $R_1 = H$; $n = 0$; $R_2 = -(CH_2)_{15}-CH_3$
On opère selon le procédé de l'exemple 4 mais en utilisant comme solvant un mélange de toluène et de diméthylformamide 40-2 (vol/vol).
Après chauffage à 100°C pendant 20 h on obtient l'ester correspondant qui est hydrolysé par chauffage au reflux pendant 15 h avec HCl 12 N. L'acide brut dissous dans l'ammoniaque fournit un monosel d'ammonium; F.: 103-110°C.
De la même façon en faisant varier le disulfure utilisé on obtient l'acide décylthiométhylènediphosphonique isolé sous forme de disel de t-butylamine (SR 41457). F.: 170-175°C.

**EXEMPLE 13**

Acide (hydroxy-2 éthylthio)méthylènediphosphonique disel de tertiobutylamine.
(SR 41318)
(I) $R_1 = H$; $n = 0$; $R_2 = HO-CH_2CH_2-$
a) On chauffe à 70°C pendant 1 min le mélange de 35 g de di(hydroxy-2 éthyl)disulfure 80 ml de dihydro-3,4 2H pyranne et 0,1 g d'acide paratoluènesulfonique.
On refroidit à 40°C et agite pendant 10 min. On verse dans 200 ml d'eau et extrait avec de l'éther. On sèche la solution éthérée et évapore le solvant à siccité pour obtenir 71 g du disulfure ayant ses deux fonctions alcool bloquées sous forme d'éther de dihydro-3,4 2H pyrannyle.
b) On effectue la condensation de disulfure ci-dessus sur le méthylènediphosphonate de tétraisopropyle selon la technique de l'exemple 5 en maintenant à 20°C pendant 3 h.
c) Le produit obtenu au paragraphe précédent (18 g) est dissous dans 150 ml de méthanol et on ajoute 2 ml d'acide chlorhydrique concentré. On chauffe au reflux 1 h, puis concentre le méthanol et verse dans 200 ml d'eau. On extrait deux fois au chlorure de méthylène, sèche la solution et concentre à siccité. On obtient ainsi 13 g de l'ester déprotégé.
d) On saponifie l'ester comme indiqué dans l'exemple 4 en chauffant au reflux avec de l'acide chlorhydrique 6N pendant 16 h.
L'acide ainsi obtenu traité par la tertiobutylamine dans l'éthanol conduit au disel SR 41318; F.: 168°C.

**EXEMPLE 14**

Acide méthylthio-méthylènediphosphonique disel de sodium.
(SR 41553)
(I) $R_1 = H$; $n = 0$; $R_2 = CH_3$
On dissout 5 g d'acide méthylthiométhylènediphosphonique (exemple 4) dans 50 ml d'eau distillée contenant 1,8 g de soude en solution.
On filtre la solution, puis on ajoute 200 ml de méthanol et laisse cristalliser. On essore le précipité et on le lave avec du méthanol. On sèche à 80°C sous vide et obtient ainsi le disel de sodium cristallisant avec 1/2 molécule d'eau; F. > 270°C.

**EXEMPLE 15**

Acide benzothiazolyl-2 thiométhylènediphosphonique trisel de tertiobutylamine.
(SR 41481)

(I) $R_1 = H$; $n = 0$; $R_2 =$ 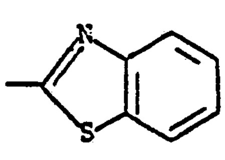

a) di(benzothiazolyl-2)disulfure.
A une solution de 30 g de mercapto-2 benzothiazole et 1,6 g de soude dans 800 ml d'éthanol absolu, on ajoute goutte à goutte la solution de 20 g d'iode et 115 g d'iodure de potassium dans 400 ml d'eau. Après la fin de l'addition, on agite 30 min à température ambiante, puis on essore le précipité qu'on lave avec de l'éthanol puis avec de l'ester.

Après séchage on obtient 28 g de disulfure; F.: 174° C.

b) On condense le disulfure ci-dessus sur le méthylènediphosphonate de tétraméthyle selon la technique de l'exemple 5 (chauffage 2 h à 20° C).

c) A une solution de 6,5 g de l'ester tétraméthylique obtenu ci-dessus dans 80 ml de tétrachlorure de carbone, on ajoute, sous atmosphère d'azote, à une température de 10° C, 7 ml de bromure de triméthylsilyle et on agite 30 min à cette température.

On ajoute 10 ml d'eau, sépare la phase aqueuse et lave celle-ci avec de l'éther. Par évaporation de la phase aqueuse, on obtient 4 g de l'acide attendu. Celui-ci est transformé en trisel par action de la t-butylamine dans l'éthanol; F. 202-204° C.

De la même façon, en utilisant le di(thiényl-2) disulfure, on obtient finalement l'acide thiényl-2 thiométhylènediphosphonique isolé sous forme de trisel de tertiobutylamine cristallisant avec 2 molécules d'eau (SR 41549); F. 210° C.

## EXEMPLE 16

Acide (méthylthio)-4 butylidènediphosphonique-1,1 sel de t-butylammonium.
(SR 41177)
(I) $R_1 = H$; $n = 3$; $R_2 = CH_3$

a) Butène-3 ylidiènediphosphonate-1,1 de tétraisopropyle.

Dans un mélange de 6,8 g de méthylènediphosphonate de tétraisopropyle et 20 ml de toluène, on ajoute, à 10° C, en 5 min, 0,5 g d'hydrure de sodium. On agite à 15° C pendant 1 h, puis introduit 15,5 ml de bromure d'allyle et agite à 20° C durant 20 h.

On reprend le mélange réactionnel dans 100 ml d'éther isopropylique et 50 ml d'eau. On décante la phase organique qu'on lave trois fois avec 50 ml d'eau, puis sèche et évapore à siccité. On obtient une huile (4,5 g) qu'on distille sous vide; E./0,02 mm: 108-110° C.

b) (Méthylthio)-4 butylidènediphosphonate-1,1 de tétraisopropyle.

On chauffe à 130° C durant 20 h dans un autoclave le mélange de 4,5 g du produit obtenu ci-dessus, 50 ml de méthylmercaptan et 0,1 g de peroxyde de benzoyle.

Après la fin de la réaction, on distille le mélange réactionnel sous vide très poussé et on recueille la fraction (3,6 g) qui distille entre 120 et 130° C sous $2 \times 10^{-5}$ mm de mercure.

c) On chauffe à reflux durant 14 h un mélange de 3,6 g de l'ester obtenu ci-dessus et 14 ml d'acide chlorhydrique 8N. On évapore à siccité sous vide et reprend le résidu dans 30 ml d'eau et 30 ml d'éther.

On sépare la phase aqueuse on la traite au charbon actif et évapore à nouveau à siccité. Le résidu repris dans 20 ml d'éthanol est traité par 2,5 ml de tertiobutylamine. Par addition de 20 ml d'éther, on obtient 1,4 g du sel attendu; F. 190° C.

## EXEMPLE 17

Acide mercapto-5 pentylidènediphosphoniquie-1,1, disel de t-butylamine
(SR 41527)

En opérant comme dans l'exemple 4 à partir de méthylènediphosphonate de tétraéthyle et d'acétylthio-4 bromo-1 butane, on prépare l'acétylthio-5 pentylidènediphosphonate-1,1 de tétraéthyle sous forme d'une huile.

L'ester ainsi obtenu est traité par HCl 12N pendant 7 h comme indiqué dans l'exemple 9. On hydrolyse ainsi les fonctions esters et le groupe acétyle du thiol pour arriver au composé SR 41527 isolé sous forme de disel de t-butylamine; F. 160° C.

## EXEMPLE 18

Acide (méthyl-1 imidazolyl-2 thio)-7 heptylidènediphosphonate-1,1, disel de tertiobutylamine.
(SR 42132)

a) On prépare le bromo-7 heptylidènediphosphonate-1,1 de tétraisopropyle ainsi qu'indiqué dans l'exemple 16 a), en utilisant le dibromo-1,6 hexane.

b) On chauffe à 80° C pendant 2 h la solution de 10 g du dérivé bromé précédent, 2 g de mercapto-2 méthyl-1 imidazole et 0,8 g de soude dans 50 ml d'éthanol à 96.

On évapore à siccité reprend le résidu dans l'eau et extrait avec de l'éther. On lave la solution avec une solution de soude 3N, puis avec de l'eau. On sèche la solution sur sulfate de sodium et évapore à siccité.

On chromatographie sur colonne de silice en éluant avec le mélange chlorure de méthylène-méthanol 98-2 vol/vol. On obtient ainsi 4 g de l'ester attendu.

c) On hydrolyse par l'acide chlorhydrique 12N en chauffant au reflux pendant 16 h. On isole le composé attendu sous forme de disel de tertiobutylamine; F. 158-162° C.

**EXEMPLE 19**

(Fluoro-4 phénylthio)-5 hydroxy-1 pentylidènediphosphonate-1,1 de tétraéthyle.
(SR 41906).

A 12,1 g de chlorure de (fluoro-4 phénylthio)-5 valéryle placé sous atmosphère d'azote, on ajoute à 30° C sous agitation 8,8 g de triéthylphosphite. On agite pendant 3 h à 35° C puis on refroidit le mélange réactionnel à 0° C et introduit en 10 min un mélange de 0,45 ml de dibutylamine et 6,5 ml de diéthylphosphite. On laisse sous agitation à 5° C pendant 1 h, puis on ajoute 20 ml d'une solution d'acide chlorhydrique 1N et 100 ml d'éther éthylique. Après agitation, on sépare la couche éthérée et réextrait la phase aqueuse avec 100 ml de chlorure de méthylène. On réunit les extraits organiques, sèche sur sulfate de sodium et évapore les solvants sous vide.

On chromatographie le résidu sur colonne de silice en éluant avec un mélange chlorure de méthylène-éthanol 95-5 vol/vol.

On obtient ainsi le produit attendu sous forme d'une huile incolore (8,7 g).

Chromatographie en couche mince de silice:

Rf: 0,40 (acétate d'éthyle-éthanol 95-5 vol/vol).

De la même façon mais en faisant varier le chlorure d'acide utilisé et/ou le phosphite, on prépare les produits (I) réunis dans le tableau ci-après.

**TABLEAU V**

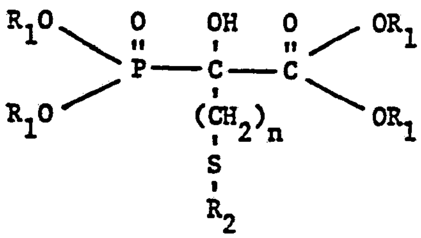

| n° de code | $R_1$ | $R_2$ | n | Caractéristiques CCM sur silice |
|---|---|---|---|---|
| SR 41962 | $-CH_3$ | $-(CH_2)_4-CH_3$ | 3 | Huile incolore Rf = 0,40 (acétate d'éthyle-éthanol, 80-20, vol/vol) |
| SR 41963 | $-C_2H_5$ | 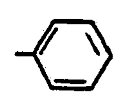 | 2 | Huile incolore Rf = 0,3 (acétate d'éthyle-éthanol, 95-5, vol/vol) |
| SR 42292 | $-C_2H_5$ | 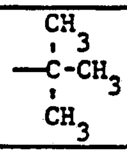 | 1 | Huile incolore Rf = 0,3 (acétate d'éthyle-éthanol, 95-5, vol/vol) |
| SR 41940 | $-C_2H_5$ | 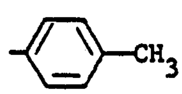 | 4 | Huile incolore Rf : 0,5 (acétate d'éthyle-éthanol, 90-10, vol/vol) |
| SR 41876 | $-C_2H_5$ | 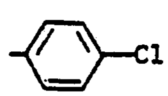 | 3 | Huile Rf : 0,34 (acétate d'éthyle-éthanol, 95-5, vol/vol) |
| SR 42295 | $-C_2H_5$ | 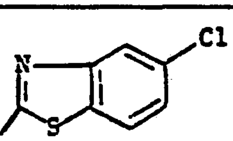 | 4 | Huile incolore Rf : 0,25 (acétate d'éthyle-éthanol, 95-5, vol/vol) |

| n° de code | $R_1$ | $R_2$ | n | Caractéristiques CCM sur silice |
|---|---|---|---|---|
| SR 42014 | $-C_2H_5$ | $-(CH_2)_2-CH_3$ | 5 | Huile incolore Rf : 0,30 (acétate d'éthyle-éthanol, 95-5, vol/vol) |
| SR 42089 | $-C_2H_5$ | $CH_3$ | 10 | Huile incolore Rf : 0,25 (acétate d'éthyle-éthanol, 95-5, vol/vol) |

**EXEMPLE 20**

(Pyridyl-2 thio)-5 hydroxy-1 pentylidènediphosphonate-1 de tétraéthyle.
(SR 42090)

a) On prépare le bromo-5 hydroxy-1 pentylidènediphosphonate de tétraéthyle selon la technique de l'exemple 19 en utilisant le chlorure de bromo-5 valéryle comme chlorure de l'acide.

On isole ce produit sous forme d'huile.

Chromatographie sur couche mince de silice:
Rf: 0,4 (acétate d'éthyle-éthanol 80-20 vol/vol).

b) A la solution de 4,4 g du produit précédent dans 5 ml d'acétonitrile refroidie à 0° C, on ajoute en 15 min le mélange de 2,2 g de mercapto-2 pyridine et 2 g de diaza-1,5 bicyclo(5.4-0)undécène-5 dans 6 ml d'acétonitrile. On agite 1 h à 0°C et chromatographie sur colonne de silice (150 g). Après lavage avec du chlorure de méthylène, on élue par un mélange chlorure de méthylène-éthanol 95-5 vol/vol.

On obtient le produit attendu sous forme d'huile jaunâtre.

Chromatographie en couche mince de silice:
Rf: 0,15 (acétate d'éthyle-éthanol 95-5 vol/vol).

De la même façon mais en remplaçant au paragraphe a) le chlorure de bromo-5 valéryle par le chlorure de bromo-6 hexanoyle on obtient le bromo-6 hydroxy-1 hexylidène-phosphonate-1,1 de diéthyle.

Celui-ci traité comme indiqué au paragraphe b) par le chloro-5 mercapto-2 benzothiazole conduit au [(chloro-5 benzothiazolyl-2)thio]-6 hydroxy-1 hexylidènediphosphonate-1,1 de tétraéthyle (SR 42294) sous forme d'un solide incolore; F. 72° C (éther isopropylique).

**EXEMPLE 21**

Acide (chloro-4 phénylthio)-4 hydroxy-1 butylidènediphosphonique-1,1 disel de tertiobutylamine.
(SR 41903)

On chauffe au reflux sous azote pendant 12 h le mélange de 5 g de (chloro-4 phénylthio)-4 hydroxy-1 butylidènediphosphonate-1,1 de tétraéthyle et 50 ml d'acide chlorhydrique 12N. On évapore à siccité sous vide, puis on dissout le résidu dans 100 ml d'eau distillée et lave deux fois avec 100 ml d'éther. On évapore la phase aqueuse à siccité. Il reste un solide incolore qu'on dissout dans 30 ml d'isopropanol et traite par 3 6 ml de tertiobutylamine. On essore le précipité et on le lave avec de l'isopropanol, de l'acétone et enfin avec de l'éther. Après séchage sous vide, on obtient un solide incolore (4 g); F. 202-205° C (déc.).

De la même façon à partir de l'ester SR 42090 (exemple 20) on obtient l'acide (pyridyl-2 thio)-5 pentylidène diphosphonique-1,1 isolé sous forme d'acide libre (SR 42099); F. 194-7° C (isopropanol).

**EXEMPLE 22**

Acide (fluoro-4 phénylthio)-5 hydroxy-1 pentylidènediphosphonique-1,1 disel de tertiobutylamine.
(SR 41909)

A la solution agitée et refroidie à 10°C de l'ester SR 41906 (exemple 19) dans 15 ml de tétrachlorure de carbone, on ajoute 5 ml de triméthybromosilane et laisse 40 h à 25° C. On ajoute 40 ml d'eau et agite pendant 1 h. On décante la phase aqueuse qu'on lave deux fois avec 30 ml de chlorure de méthylène. On évapore la phase aqueuse à siccité sous vide. On reprend le résidu dans 30 ml d'eau et évapore à nouveau à siccité sous vide.

Le résidu sirupeux est transformé en sel de dibutylamine comme indiqué à l'exemple 21. On obtient finalement un solide incolore; F. 211-5°C.

De la même façon à partir de différents esters obtenus précédemment on obtient les acides figurant dans le tableau suivant.

**TABLEAU VI**

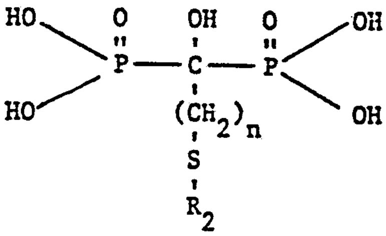

| n° de code | n | $R_2$ | Forme d'isolement<br>Caractéristiques |
|---|---|---|---|
| SR 42142 | 10 | 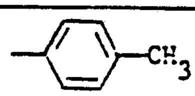 | disel de tertiobutylamine<br>F. : 242-246°C |
| SR 42097 | 10 | $-CH_3$ | disel de tertiobutylamine<br>F. : 195-200°C (déc.) |
| SR 42304 | 5 | 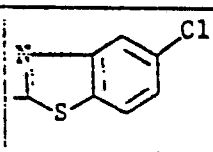 | acide libre<br>cristallise avec<br>1 molécule d'eau<br>F. : 205-208°C |
| SR 42015 | 5 | $-CH_2CH_2CH_3$ | sel avec 2,5 molécules<br>de tertiobutylamine<br>F. : 202-205°C (déc.) |
| SR 42296 | 4 | 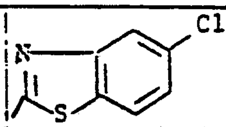 | acide libre<br>F. : 165-168°C |
| SR 42016 | 4 | 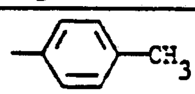 | disel de sodium<br>F. > 300°C |
| SR 42293 | 1 | 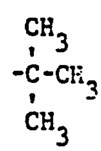 | sel de tertiobutylamine<br>(1,75 mole)<br>F. : 220-225°C (déc.) |
| SR 41961 | 3 | $-(CH_2)_4-CH_3$ | sel de tertiobutylamine<br>(1,5 mole)<br>F. : 175-178°C |
| SR 42017 | 2 | 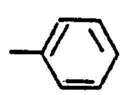 | acide libre<br>F. : 145-148°C |

Les composés selon l'invention sont avantageusement utilisés comme médicaments anti-inflammatoires et antirhumatismaux et leurs propriétés pharmacologiques ont été mises en évidence de la manière suivante.

**ETUDE IN VITRO**

L'étude in vitro est basée sur le fait que des chondrocytes en culture sécrètent des protéinases neutres après stimulation par un facteur synthétisé par des macrophages péritonéaux et par les cellules mononucléées du sang.

L'implication de ce type de stimulation dans les maladies rhumatismales a été clairement démontrée dans de nombreuses publications.

Le principe du test consiste donc à étudier la sécrétion de protéinases neutres par les chondrocytes stimulés traités par les produits en comparaison avec la sécrétion des chondrocytes stimulés non traités.

Le protocole suivant a été utilisé.

**Préparation des chondrocytes.**

Les chondrocytes sont isolés à partir de cartilage de septum nasal de veau ou de cartilage articulaire de lapin, par digestion enzymatique et sont mis en culture dans du milieu DMEM à 10 % de sérum de veau, à une densité de $5 \times 10^{15}$ cellules par boîte de Petri de 10 cm. Le milieu de culture est renouvelé toutes les 48 h.

Après une semaine de culture les cellules sont sub-cultivées par traitement à la trypsine EDTA. Les cellules sont remises en culture dans du milieu DMEM à 10 % de sérum de veau foetal, à une densité de $2 \times 10^5$ cellules par puits de 16 mm. La confluence est réalisée après 3 jours de culture.

**Préparation du milieu conditionné de macrophages péritonéaux.**

Les macrophages péritonéaux de lapin sont obtenus selon la méthode décrite par DESHMUKH-PHADKE et coll. Biochemical Biophysical Research Communication 85 490-6 (1978).

Les cellules mononucléées de sang humain sont préparées et cultivées suivant la procédure de DAYER et coll. [The Journal of Immunology 124, 1712-1720 (1980)].

Après culture le milieu contenant le "chondrocyte stimulating factor" est récupéré, filtré sur Millipore 0,22 µm et congelé à -20°C.

**Stimulation de chondrocytes.**

A confluence, le milieu de culture des chondrocytes est changé par du milieu DMEM à 1 % de sérum de veau foetal contenant 20 % de milieu contenant le facteur d'activation des chondrocytes.

Les produits à tester sont introduits dans le milieu de culture en même temps que le milieu d'activation à une concentration évitant la cytotoxicité du produit ou son insolubilité. Après trois jours de culture, le milieu est récupéré en vue du dosage de son contenu en protéinases neutres.

**Dosage des protéinases neutres.**

On utilise le protocole de VAES et coll. Biochemical Journal, 172, 261 (1978).

Les résultats obtenus avec divers produits de l'invention sont réunis dans le tableau 7. Ils sont exprimés en pour cent d'inhibition de la sécrétion de protéinases neutres par les chondrocytes stimulés traités par les produits à étudier par rapport à celle de chondrocytes stimulés non traités par les produits.

**TABLEAU VII**

| n° de code du produit | Chondrocyte en culture % d'inhibition | n° de code de produit | Chondrocyte en culture % d'inhibition |
|---|---|---|---|
| SR 41036 | 72 ± 8 | SR 41319 | 85 ± 1 |
| SR 41100 | 20 ± 1 | SR 41179 | 75 ± 7 |
| SR 41421 | 97 ± 2 | SR 41264 | 50 ± 10 |
| SR 41457 | 95 ± 3 | SR 41263 | 90 ± 1 |
| SR 41453 | 40 ± 2 | SR 41480 | 56 ± 9 |
| SR 41266 | 68 ± 8 | SR 41481 | 74 ± 9 |
| SR 41344 | 31 ± 6 | SR 41177 | 69 ± 4 |
| SR 41482 | 83 ± 6 | SR 41342 | 99 ± 1 |
| SR 41456 | 98 ± 2 | SR 41388 | 90 ± 1 |
| SR 41908 | 82 ± 2 | SR 41960 | 55 ± 4 |
| SR 41689 | 90 ± 3 | SR 41690 | 97 ± 1 |
| SR 41688 | 94 ± 1 | SR 42249 | 92 ± 4 |
| SR 41549 | 58 ± 4 | SR 41687 | 89 ± 2 |
| SR 41527 | 54 ± 3 | SR 41959 | 79 ± 2 |
| SR 41625 | 92 ± 0 | SR 42099 | 50 ± 5 |

**ETUDE IN VIVO** : Arthrite à l'adjuvant.

L'injection de Mycobacterium chez le rat entraîne une polyarthrite rappelant par certains aspects l'arthrite rhumatoïde humaine.

**Protocole.**

Une suspension de Mycobacterium tuberculosis (0,4 mg pour 0,05 ml d'huile de paraffine) est injectée par voie intradermique dans la queue de rats mâles Sprague-Dawley d'un poids moyen de 150 g.

Après 15 jours, on trie les animaux présentant les symptômes d'arthrite les plus marqués. Ces rats sont répartis en lots de 5 animaux, puis chaque groupe est traité par le produit à étudier à la dose de 10 mg/kg par jour, pendant 6 jours par semaine par voie cutanée. Un des groupes ne reçoit que le solvant et sert de témoin.

Après 3 semaines de traitement les animaux sont sacrifiés et la patte arrière droîte est sectionnée au niveau de l'articulation tibiotarsienne, puis pesée.

Pour chaque groupe on détermine la moyenne et l'erreur standard de ces poids.

L'activité de chaque produit est exprimée par la variation en pour cent du poids moyen des pattes des rats arthritiques traités par rapport à celui des pattes arthritiques des rats témoins.

Les résultats obtenus avec divers produits de l'invention sont réunis dans le tableau 8.

**TABLEAU VIII**

| n° de code du produit | Arthrite à l'adjuvant Poids de la patte % d'inhibition | n° de code du produit | Arthrite à l'adjuvent Poids de la patte % d'inhibition |
|---|---|---|---|
| SR 41036 | 23 | SR 41319 | 29 |
| SR 41100 | 37 | SR 41452 | 15 |
| SR 41421 | 55 | SR 41272 | 25 |
| SR 41454 | 33 | SR 41179 | 25 |
| SR 41453 | 58 | SR 41264 | 43 |
| SR 41266 | 29 | SR 41263 | 19 |
| SR 41344 | 35 | SR 41273 | 39 |
| SR 41388 | 42 | SR 41552 | 36 |
| SR 41482 | 41 | SR 41689 | 31 |
| SR 41690 | 35 | SR 41319 | 35 |
| SR 41688 | 38 | SR 41549 | 34 |
| SR 41480 | 40 | SR 41481 | 40 |
| SR 41342 | 38 | SR 41687 | 38 |
| SR 41527 | 34 | SR 41959 | 60 |
| SR 41341 | 24 | SR 42017 | 49 |
| SR 41961 | 53 | SR 41903 | 55 |
| SR 41903 | 55 | SR 42016 | 48 |
| SR 41909 | 53 | SR 42099 | 44 |
| SR 42015 | 47 | SR 42097 | 50 |

Par ailleurs, les produits selon l'invention sont peu toxiques.

Ils peuvent être utilisés en thérapeutique humaine pour le traitement des affections dues à des phénomènes inflammatoires et en particulier pour le traitement des états arthritiques. Notamment les composés selon l'invention peuvent être utilisés dans le traitement de la polyarthrite rhumatoïde.

Les composés selon l'invention peuvent être présentés sous les formes appropriées à l'administration par les voies orale endorectale et parentérale.

Il peut s'agir notamment de gélules ou de comprimés contenant une quantité de principe actif de 10 à 500 mg/unité.

La posologie quotidienne de ces produits chez l'adulte peut être de l'ordre de 100 mg à 5 g par jour répartis en plusieurs prises.

A titre d'exemple on peut donner la composition galénique suivante:

Gélules

| | |
|---|---|
| CM® 41421 | 200 mg |
| Aerosil | 1 mg |
| Stéarate de magnésium | 3 mg |
| Amidon STARX® 1500 | 96 mg |
| | 300 mg |

**Revendications** pour les Etats Contractants BE, CH, DE, FR, GB, IT, LI, LU, NL, SE.

1. Dérivés de l'acide méthylènediphosphonique de formule

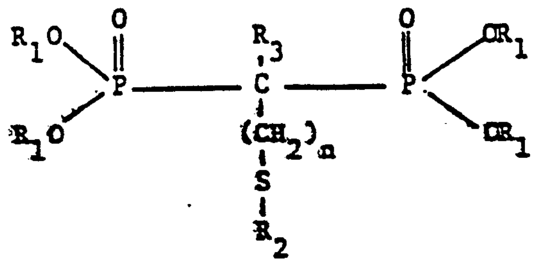

(I)

dans laquelle:

- $R_1$ représente l'hydrogène ou un groupe alkyle inférieur droit ou ramifié ayant de 1 à 4 atomes de carbone;
- $R_2$ représente:
- l'hydrogène
- un groupe alkyle éventuellement substitué par un groupe hydroxyle, thiol, un ou plusieurs atomes d'halogène un groupe alcoxycarbonyle ou un groupe

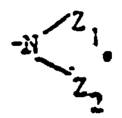

où $Z_1$ et $Z_2$ considérés indépendamment représentent l'hydrogène ou un groupe alkyle inférieur;

- un groupe phényle éventuellement substitué une ou plusieurs fois par un halogène un groupe nitro un groupe alkyle inférieur, alcoxy inférieur, trifluorométhyle ou encore par un groupe $NH_2$ ou un groupe COOH ou COO alkyle;
- un groupe

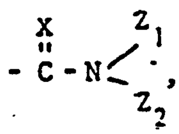

où X désigme l'oxygène ou le soufre et $Z_1$ et $Z_2$ ont été définis ci-dessus;

- un hétérocycle à 5 ou 6 chaînons comportant 1 ou 2 hétéroatomes choisis parmi l'azote et le soufre;
- un hétérocycle à 5 chaînons condensé avec un noyau benzénique et répondant à la formule:

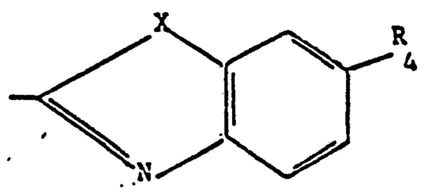

où X peut représenter l'oxygène, un groupe NH ou le soufre et $R_4$ désigne l'hydrogène ou un atome d'halogène de préférence le chlore;

- $R_3$ désigne l'hydrogène ou un groupe hydroxyle,
- n représente un nombre entier compris entre 0 et 10 à la condition que:
- n soit différent de zéro lorsque $R_3$ = OH;
- $R_2$ soit différent des groupes méthyle ou pyridyle lorsque $R_1 = R_3$ = H et n = 0;
- $R_2$ soit différent du groupe de formule:

10 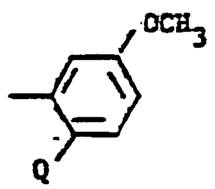

dans lequel Q est un atome d'halogène un groupe nitro ou un groupe trifluorométhyle lorsque $R_1$ est le groupe méthyle ou éthyle, $R_3$ = H et n = 0,

- $R_2$ soit différent du groupe phényle lorsque $R_1$ est le groupe éthyle, $R_3$ = H et n = 0,
- $R_2$ n'est pas un groupe alkyle ou phényle non substitués ou un groupe acétoxy lorsque $R_3$ est H et n = 1,
- $R_2$ n'est pas le groupe méthyle lorsque $R_3$= OH et n = 1; ainsi que les sels desdits dérivés dans lesquels $R_1$ = H avec des bases minérales et organiques.

2. Dérivés de l'acide méthylènediphosphonique de formule

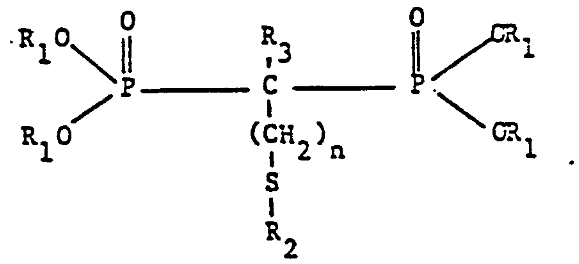

(I)

dans laquelle:

- $R_1$ représente l'hydrogène ou un groupe alkyle inférieur droit ou ramifié ayant de 1 à 4 atomes de carbone;
- $R_2$ représente:
- l'hydrogène
- un groupe alkyle éventuellement substitué par un groupe hydroxyle, thiol, un ou plusieurs atomes d'halogène, un groupe alcoxycarbonyle ou un groupe

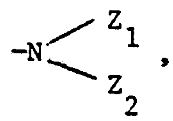

ou $Z_1$ et $Z_2$ considérés indépendamment représentent l'hydrogène ou un groupe alkyle inférieur:

- un groupe phényle éventuellement substitué une ou plusieurs fois par un halogène, un groupe nitro, un groupe alkyle inférieur alcoxy inférieur, trifluorométhyle ou encore par un groupe $NH_2$ ou un groupe COOH ou COO alkyle;
- un groupe

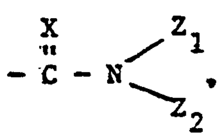

où X désigne l'oxygène ou le soufre, et $Z_1$ et $Z_2$ ont été définis ci-dessus;

- un hétérocycle à 5 ou 6 chaînons comportant 1 ou 2 hétéro-atomes choisis parmi l'azote et le soufre;
- un hétérocycle à 5 chaînons condensé avec un noyau benzénique et répondant à la formule:

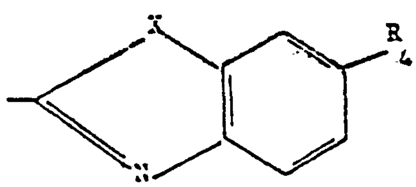

où X peut représenter l'oxygène un groupe NH ou le souire et $R_4$ désigne l'hydrogène ou un atome d'halogène de préférence, le chlore,

- $R_3$ désigne l'hydrogène ou un groupe hydroxyle,
- n représente un nombre entier compris entre 0 et 10 à la condition que n soit différent de zéro lorsque $R_3$ = OH et les sels desdits dérivés dans lesquels $R_1$ = H avec des bases minérales ou organiques pour leur utilisation comme agents anti-inflammatoires.

3. Dérivés de l'acide méthylène-diphosphonique caractérisés en ce qu'ils répondent à la formule:

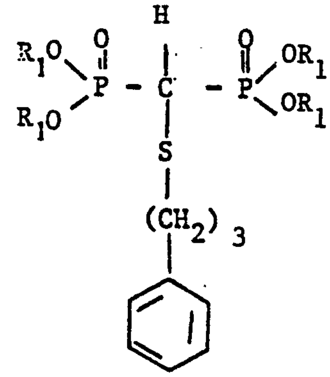

dans laquelle $R_1$ est l'hydrogène ou le groupe isopropyle.

4. Dérivés de l'acide méthylène-diphosphonique de formule

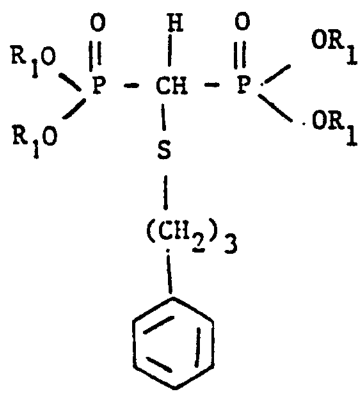

dans laquelle $R_1$ est l'hydrogène ou le groupe isopropyle pour leur utilisation comme agents anti-inflammatoires.

5. Dérivés de l'acide méthylène-diphosphonique caractérisé en ce qu'il répond à la formule:

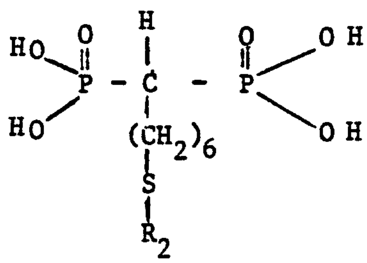

dans laquelle $R_2$ est le groupe méthyl-1 imidazol-2-yle.

6. Dérivé de l'acide méthylènediphosphonique caractérisé en ce qu'il répond à la formule:

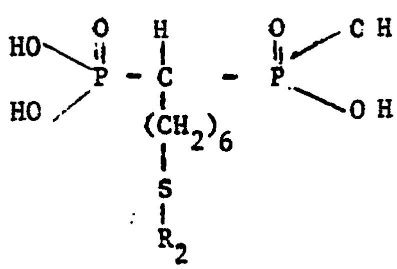

dans laquelle $R_2$ est le groupe méthyl-1 imidazol-2-yle pour son utilisation comme agent anti-inflammatoire.

7. Procédé de préparation de produits selon l'une des revendications 1 à 6 dans lesquels $R_3$ est l'hydrogène, caractérisé en ce que l'on utilise comme produit de départ un ester d'alkyle inférieur d'acide méthylènediphosphonique que l'on transforme en dérivé métallique correspondant par action d'un agent de sodation tel que l'hydrure de sodium ou par action de la potasse au sein du toluène, puis on fait réagir ledit dérivé métallique sur un composé choisi parmi les produits de formule $R_2$-S-S-$R_2$, $R_2$SCl et Hal-$(CH_2)_n$-S-$R_2$ dans laquelle Hal est un halogène de façon à aboutir à un produit de formule (I) puis éventuellement ledit produit de formule (I), qui est sous forme d'ester est saponifié et éventuellement transformé en l'un de ses sels.

8. Procédé selon la revendication 7, caractérisé en ce que le dérivé sodé est mis en réaction avec $R_2$-S-S-$R_2$ dans un solvant tel que le toluène ou le diméthylformamide à une température comprise entre 20°C et la température d'ébullition du solvant.

9. Procédé selon la revendication 7, caractérisé en ce que le dérivé sodé est mis en réaction avec Hal-$(CH_2)$n-S-$R_2$ dans un solvant tel que le toluène et le diméthylformamide à une température comprise entre 20°C et la température d'ébullition du solvant.

10. Procédé de préparation de produits selon la revendication 1 dans lesquels n est égal à 3, caractérisé en ce que l'on utilise comme produit de départ un ester d'alkyle inférieur de l'acide méthylènediphosphonique que l'on fait réagir sur un halogénure d'allyle, puis en ce que l'on fait réagir un thiol de formule $SHR_2$ sur le produit obtenu et en ce qu'éventuellement on saponifie le produit obtenu, et on le transforme en l'un de ses sels.

11. Procédé pour l'obtention des produits selon la revendication 1 dans laquelle $R_3$ est l'hydroxyle, caractérisé en ce qu'il consiste à faire réagir un chlorure d'acide de formule $R_2S$-$(CH_2)_n$-COCl dans laquelle $R_2$ est tel que défini dans la revendication 1 avec un trialkylphosphite à une température comprise entre 20 et 50°, à traiter le produit obtenu par un dialkylphosphite en présence d'une amine secondaire à une température comprise entre 0 et 20°C, éventuellement à saponifier le produit obtenu et à le transformer en l'un de ses sels.

12. Procédé pour l'obtention des produits selon la revendication 1, dans laquelle $R_3$ est l'hydroxyle, caractérisé en ce qu'il consiste à faire réagir un composé de formule:

Hal-$(CH_2)_n$-COCl avec un trialkylphosphite à une température comprise entre 20 et 50°C, à traiter le produit obtenu par un dialkylphosphite en présence d''une amine secondaire à une température comprise entre 0 et 20°C, et à faire réagir le composé obtenu de formule:

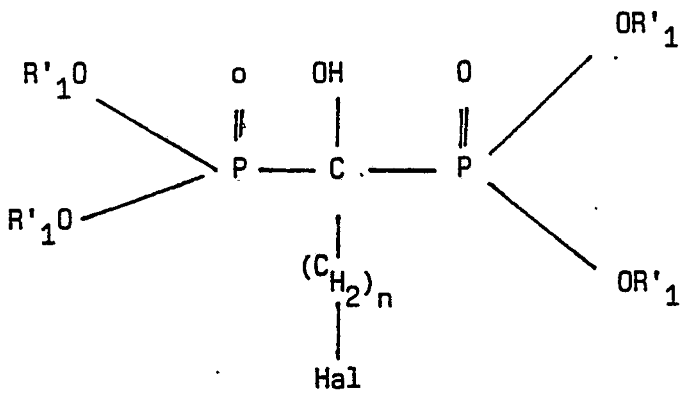

avec un thiol $R_2SH$ dans un solvant tel que l'acétonitrile et en présence de diaza-1,5 bicyclo (5,4,0) undecène-5, éventuellement à saponifier le produit obtenu et à le transformer en l'un de ses sels.

13. Médicaments pour l'utilisation à titre d'agents antiinflammatoires caractérisés en ce qu'ils contiennent un produit selon l'une des revendications 2 ou 3.

14. Médicaments selon la revendication 10, caractérisés en ce qu'ils contiennent de 10 à 500 mg d'un produit selon la revendication 2.

**Revendications** pour l'Etat Contractant: AT.

1. Procédé pour l'obtention des dérivés de l'acide méthylènediphosphonique de formule:

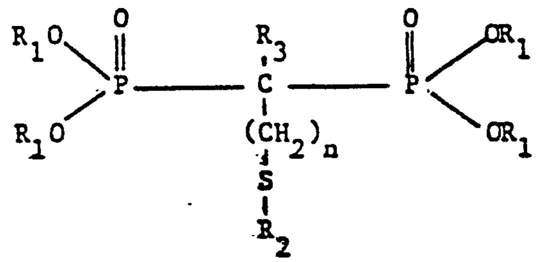

(I)

- $R_1$ représente l'hydrogène ou un groupe alkyle inférieur droit ou ramifié ayant de 1 à 4 atomes de carbone;
- $R_2$ représente:
- l'hydrogène;
- un groupe alkyle éventuellement substitué par un groupe hydroxyle, thiol, un ou plusieurs atomes d'halogène un groupe alcoxycarbonyle ou un groupe

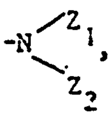

où $Z_1$ et $Z_2$ considérés indépendamment représentent l'hydrogène ou un groupe alkyle inférieur;
- un groupe phényle éventuellement substitué une ou plusieurs fois par un halogène un groupe nitro un groupe alkyle inférieur alcoxy inférieur trifluorométhyle ou encore par un groupe $NH_2$ ou un groupe COOH ou COO alkyle;
- un groupe

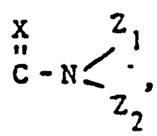

où X désigne l'oxygène ou le soufre, et $Z_1$ et $Z_2$ ont été définis ci-dessus;
- un hétérocycle à 5 ou 6 chaînons comportant 1 ou 2 hétéroatomes choisis parmi l'azote et le soufre;
- un hétérocycle à 5 chaînons condensé avec un noyau benzénique et répondant à la formule:

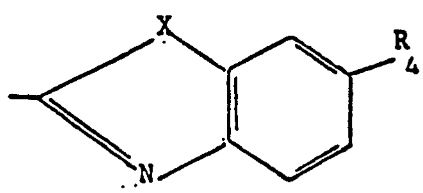

où X peut représenter l'oxygène, un groupe NH ou le soufre et $R_4$ désigne l'hydrogène ou un atome d'halogène de préférence le chlore,
- $R_3$ désigne l'hydrogène,
- n représente un nombre entier compris entre 0 et 10 à la condition que:
- $R_2$ soit différent des groupes méthyle ou pyridyle lorsque $R_1$ = H et n = 0;
- $R_2$ soit différent du groupe de formule:

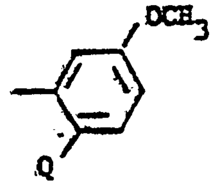

dans lequel Q est un atome d'halogène un groupe nitro ou un groupe trifluorométhyle lorsque $R_1$ est le groupe méthyle ou éthyle, et n = 0,

- $R_2$ soit différent du groupe phényle lorsque $R_1$ est le groupe éthyle, et $n = 0$;
- $R_2$ n'est pas un groupe alkyle ou phényle non substitués ou un groupe acétoxy lorsque $n = 1$,

ainsi que les sels desdits dérivés dans lesquels $R_1 = H$ avec des bases minérales et organiques, caractérisé en ce que l'on utilise comme produit de départ un ester d'alkyle inférieur d'acide méthylènedisphosphonique que l'on transforme en dérivé métallique correspondant par action d'un agent de sodation tel que l'hydrure de sodium ou par action de la potasse au sein du toluène, puis on fait agir ledit dérivé métallique sur un composé choisi parmi les produits de formule $R_2$-S-S-$R_2$, $R_2SCl$ et Hal-$(CH_2)_n$-S-$R_2$ dans laquelle Hal est un halogène de façon à aboutir à un produit de formule (I), puis éventuellement ledit produit de formule (I) qui est sous forme d'ester est saponifié et éventuellement transformé en l'un de ses sels.

2. Procédé selon la revendication 1, caractérisé en ce que le dérivé sodé est mis en réaction avec $R_2$-S-S-$R_2$ dans un solvant tel que le toluène ou le diméthylformamide à une température comprise entre 20°C et la température d'ébullition du solvant.

3. Procédé selon la revendication 1, caractérisé en ce que le dérivé sodé est mis en réaction avec Hal-$(CH_2)_n$-S-$R_2$ dans un solvant tel que le toluène et le diméthylformamide à une température comprise entre 20°C et la température d'ébullition du solvant.

4. Procédé pour l'obtention des dérivés de l'acide méthylènediphosphonique de formule:

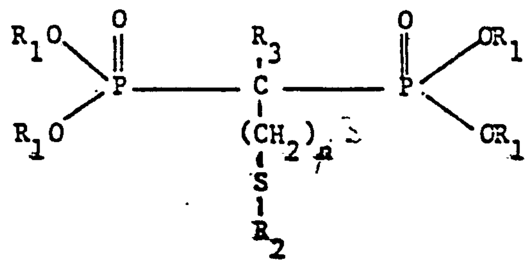

(I)

dans laquelle:
- $R_1$ représente l'hydrogène ou un groupe, alkyle inférieur droit ou ramifié ayant de 1 à 4 atomes de carbone;
- $R_2$ représente
- l'hydrogène.
- un groupe alkyle éventuellement substitué par un groupe hydroxyle thiol, un ou plusieurs atomes d'halogène un groupe alcoxycarbonyle ou un groupe

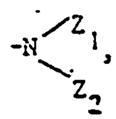

où $Z_1$ et $Z_2$ considérés indépendamment représentent l'hydrogène ou un groupe alkyle inférieur;
- un groupe phényle éventuellement substitué une ou plusieurs fois par un halogène, un groupe nitro, un groupe alkyle inférieur alcoxy inférieur trifluorométhyle ou encore par un groupe $NH_2$ ou un groupe COOH ou COO alkyle;
- un groupe

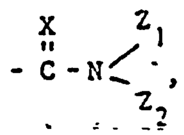

où X désigne l'oxygène ou le soufre et $Z_1$ et $Z_2$ ont été définis ci-dessus;
- un hétérocycle à 5 ou 6 chaînons comportant 1 ou 2 hétéroatomes choisis parmi l'azote et le soufre;
- un hétérocycle à 5 chaînons condensé avec un noyau benzénique et répondant à la formule:

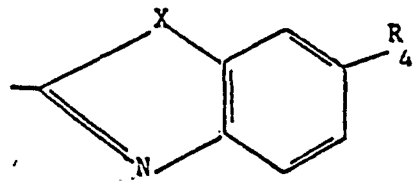

où X peut représenter l'oxygène, un groupe NH ou le soufre et $R_4$ désigne l'hydrogène ou un atome d'halogène de préférence le chlore,

$R_3$ désigne l'hydrogène ou un groupe hydroxyle, caractérisé en ce que l'on utilise comme produit de départ

un ester d'alkyle inférieur de l'acide méthylènediphosphonique que l'on fait réagir sur un halogénure d'allyle, puis en ce que l'on fait réagir un thiol de formule $R_2SH$ sur le produit obtenu et en ce qu'éventuellement on saponifie le produit obtenu et on le transforme en l'un de ses sels.

5. Procédé pour l'obtention des dérivés de l'acide méthylènediphosphonique de formule:

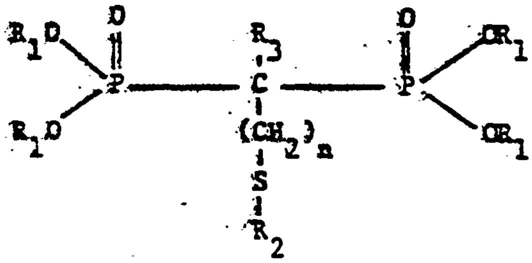

(I)

dans laquelle:

- $R_1$ représente l'hydrogène ou un groupe alkyle inférieur droit ou ramifié ayant de 1 à 4 atomes de carbone;
- $R_2$ représente
- l'hydrogène;
- un groupe alkyle éventuellement substitué par un groupe hydroxyle, thiol, un ou plusieurs atomes d'halogène un groupe alcoxycarbonyle ou un groupe

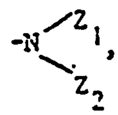

,

où $Z_1$ et $Z_2$ considérés indépendamment représentent l'hydrogène ou un groupe alkyle inférieur;

- un groupe phényle éventuellement substitué une ou plusieurs fois par un halogène, un groupe nitro un groupe alkyle inférieur alcoxy inférieur, trifluorométhyle ou encore par un groupe $NH_2$ ou un groupe COOH ou COO alkyle;
- un groupe

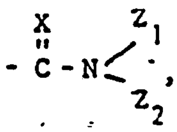

,

où X désigne l'oxygène ou le soufre et $Z_1$ et $Z_2$ ont été définis ci-dessus;

- un hétérocycle à 5 ou 6 chaînons comportant 1 ou 2 hétéroatomes choisis parmi l'azote et le soufre;
- un hétérocycle à 5 chaînons condensé avec un noyau benzénique et répondant à la formule:

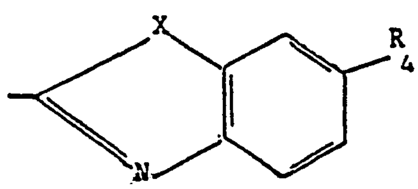

où X peut représenter l'oxygène, un groupe NH ou le soufre et $R_4$ désigne l'hydrogène ou un atome d'halogène de préférence le chlore,

- $R_3$ est un groupe hydroxyle,
- n représente un nombre entier compris entre 1 et 10 à la condition que:
- $R_2$ n'est pas le groupe méthyle lorsque $n = 1$; ainsi que les sels desdits dérivés dans lesquels $R_1 = H$ avec des bases minérales et organiques,

caractérisé en ce qu'il consiste à faire réagir un chlorure d'acide de formule $R_2S-(CH_2)_n-COCl$ dans laquelle $R_2$ est tel que défini dans la revendication 1 avec un trialkylphosphite à une température comprise entre 20 et 50°C, à traiter le produit obtenu par un dialkylphosphite en présence d'une amine secondaire à une température comprise entre 0 et 20°C, éventuellement à saponifier le produit obtenu et à la transformer en l'un de ses sels.

6. Procédé pour l'obtention des dérivés de l'acide méthylènediphosphonique tels que définis dans la revendication 5, caractérisé en ce qu'il consiste à faire réagir un composé de formule: $Hal-(CH_2)_n-COCl$ avec un trialkylphosphite à une température comprise entre 20 et 50°C, à traiter le produit obtenu par un

dialkylphosphite en présence d'une amine secondaire à une température comprise entre 0 et 20°C, à faire réagir le composé obtenu de formule :

5 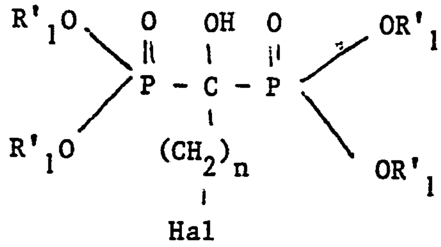

avec un thiol de formule $R_2SH$ dans un solvant tel que l'acétonitrile et en présence de diaza-1,5 bicyclo(5,4,0) undecène-5, éventuellement à saponifier le produit obtenu et à le transformer en l'un de ses sels.

7. Procédé pour l'obtention des dérivés de l'acide méthylènediphosphonique de formule

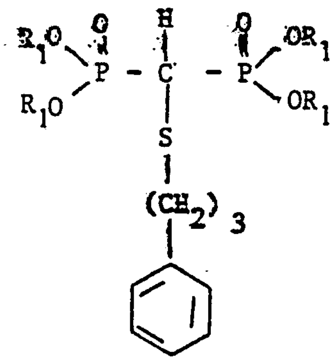

dans laquelle $R_1$ est l'hydrogène ou le groupe isopropyle, caractérisé en ce que l'on utilise comme produit de départ le méthylènediphosphonate de tétraisopropyle que l'on transforme en dérivé métallique correspondant par action de la potasse au sein du toluène, puis on fait réagir ledit dérivé métallique avec le di(phènyl-3 propyl) disulfure, puis on saponifie éventuellement ledit composé obtenu.

8. Procédé pour l'obtention des dérivés de l'acide méthylènediphosphonique de formule:

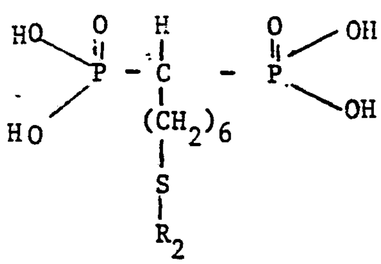

dans laquelle $R_2$ est le groupe méthyl-1 imidazol-2-yle caractérisé en ce que l'on utilise comme produit de départ le méthylènediphosphate de tétraisopropyle que l'on transforme en le dérivé métallique correspondant par action de l'hydrure de sodium, puis on fait réagir le dérivé métallique avec le dibromo-1,6 hexane, le dérivé bromé obtenu est ensuite mis à réagir avec le mercapto-2 méthyl-1 imidazole.

9. Utilisation des dérivés de l'acide méthylènediphosphonique de formule:

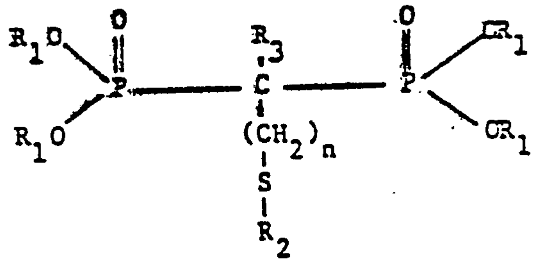

(I)

dans laquelle:

- $R_1$ représente l'hydrogène ou un groupe alkyle inférieur droit ou ramifié ayant de 1 à 4 atomes de carbone;
- $R_2$ représente:
- l'hydrogène
- un groupe alkyle éventuellement substitué par un groupe hydroxyle, thiol, un ou plusieurs atomes d'halogène, un groupe alcoxycarbonyle ou un groupe

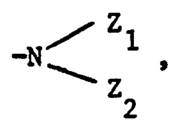

ou $Z_1$ et $Z_2$ considérés indépendamment représentent l'hydrogène ou un groupe alkyle inférieur:

- un groupe phényle éventuellement substitué une ou plusieurs fois par un halogène, un groupe nitro, un groupe alkyle inférieur, alcoxy inférieur, trifluorométhyle ou encore par un groupe $NH_2$ ou un groupe COOH ou COO alkyle;
- un groupe

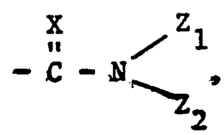

où X désigne l'oxygène ou le soufre, et $Z_1$ et $Z_2$ ont été définis ci-dessus;

- un hétérocycle à 5 ou 6 chaînons comportant 1 ou 2 hétéroatomes choisis parmi l'azote et le soufre;
- un hétérocycle à 5 chaîons condensé avec un noyau benzénique et répondant à la formule:

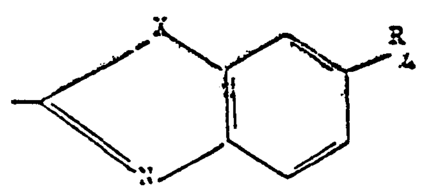

où X peut représenter l'oxygène, un groupe NH ou le soufre et $R_4$ désigne l'hydrogène ou un atome d'halogène de préférence, le chlore,

- $R_3$ désigne l'hydrogène ou un groupe hydroxyle,
- n représente un nombre entier compris entre 0 et 10 à la condition que n soit différent de zéro lorsque $R_3$ = OH et les sels desdits dérivés dans lesquels $R_1$ = H avec des bases minérales ou organiques pour la préparation de médicaments utiles à titre d'agents anti-inflammatoires.

10. Utilisation des dérivés de l'acide méthylènediphosphonique de formule

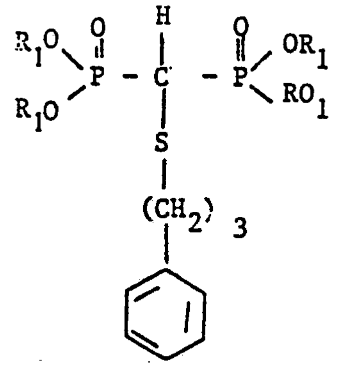

dans laquelle $R_1$ est l'hydrogène ou le groupe isopropyle, ou de formule:

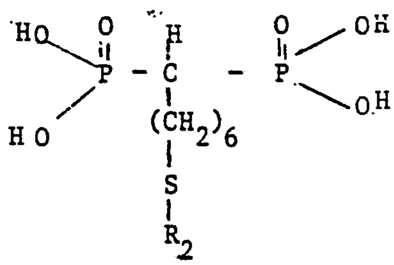

dans laquelle $R_2$ est le groupe methyl-1 imidazol-2-yle pour la préparation de médicaments utiles à titre d'agents anti-inflammatoires.

**Patentansprüche** für die Vertragsstaaten BE, CH, DE, FR, GB, IT, LI, LU, NL, SE:

1. Methylendiphosphonsäurederivate der Formel

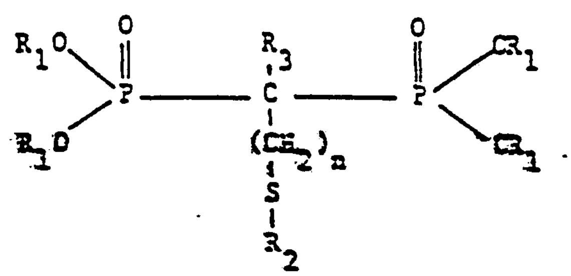

(I)

worin:

- $R_1$ Wasserstoff oder eine gerade oder verzweigte Niedrigalkylgruppe mit 1 bis 4 Kohlenstoffatomen darstellt;
- $R_2$ für
- Wasserstoff
- eine gegebenenfalls durch eine Hydroxyl-, Thiolgruppe, ein oder mehrere Halogenatome, eine Alkoxycarbonylgrupoe oder eine Gruppe

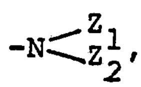,

worin $Z_1$ und $Z_2$, einzeln betrachtet, Wasserstoff oder eine Niedrigalkylgruppe darstellen, substituierte Alkylgruppe;
- eine gegebenenfalls ein- oder mehrmals durch ein Halogen, eine Nitrogruppe, eine Niedrigalkyl-, Niedrigalkoxy-, Trifluormethylgruppe oder aber eine Gruppe $NH_2$ oder eine Gruppe COOH oder COO-alkyl substituierte Phenylgruppe;
- eine Gruppe

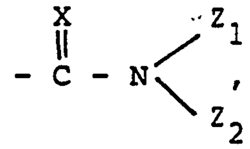

worin X Sauerstoff oder Schwefel bezeichnet und $Z_1$ und $Z_2$ obige Bedeutung haben;
- einen Heterocyclus mit 5 oder 6 Ketten, umfassend 1 oder 2 Heteroatome, ausgewählt aus Stickstoff oder Schwefel;
- einen Heterocyclus mit 5 Ketten, kondensiert mit einem Benzolkern und der Formel

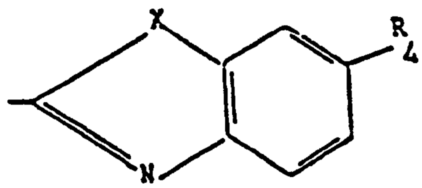

worin X Sauerstoff, eine Gruppe NH oder Schwefel sein kann und $R_4$ Wasserstoff oder ein Halogenatom, vorzugsweise Chlor, bezeichnet,
steht,
- $R_3$ Wasserstoff oder eine Hydroxylgruppe bezeichnet,
- n eine ganze Zahl zwischen 0 und 10 bedeutet, mit der Maßgabe, daß
- n ungleich Null ist, wenn $R_3 = OH$;
- $R_2$ keine Methyl- oder Pyridylgruppe ist, wenn $R_1 = R_3 = H$ und $n = 0$;
- $R_2$ keine Gruppe der Formel

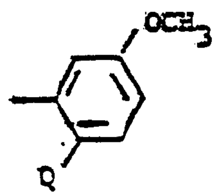

worin Q ein Halogenatom, eine Nitrogruppe oder eine Trifluormethylgruppe darstellt, ist, wenn $R_1$ eine Methyl- oder Äthylgruppe ist, $R_3 = H$ und $n = 0$;
- $R_2$ keine Phenylgruppe ist, wenn $R_1$ eine Äthylgruppe ist, $R_3 = H$ und $n = 0$;
- $R_2$ keine nichtsubstituierte Alkyl- oder Phenylgruppe oder Acetoxygruppe ist, wenn $R_3$ für H steht und $n = 1$;
- $R_2$ keine Methylgruppe ist, wenn $R_3 = OH$ und $n = 1$; sowie die Salze dieser Derivate, worin $R_1 = H$, mit Mineral- und organischen Basen.

2. Methylendiphosphonsäurederivate der Formel

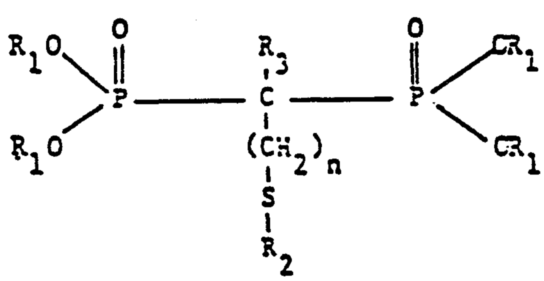

(I)

worin:
- $R_1$ Wasserstoff oder eine gerade oder verzweigte Niedrigalkylgruppe mit 1 bis 4 Kohlenstoffatomen darstellt;
- $R_2$ für
- Wasserstoff
- eine gegebenenfalls durch eine Hydroxyl-, Thiolgruppe, ein oder mehrere Halogenatome, eine Alkoxycarbonylgruppe oder eine Gruppe

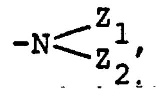

worin $Z_1$ und $Z_2$, einzeln betrachtet, Wasserstoff oder eine Niedrigalkylgruppe darstellen, substituierte Alkylgruppe;

- eine gegebenenfalls ein- oder mehrmals durch ein Halogen, eine Nitrogruppe, eine Niedrigalkyl-, Niedrigalkoxy-, Trifluormethylgruppe oder aber eine Gruppe $NH_2$ oder eine Gruppe COOH oder COO-Alkyl substituierte Phenylgruppe;
- eine Gruppe

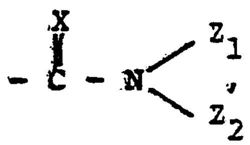

worin X Sauerstoff oder Schwefel bezeichnet und $Z_1$ und $Z_2$ obige Bedeutung haben;

- einen Heterocyclus mit 5 oder 6 Ketten, umfassend 1 oder 2 Heteroatome, ausgewählt aus Stickstoff oder Schwefel;
- einen Heterocyclus mit 5 Ketten, kondensiert mit einem Benzolkern und der Formel

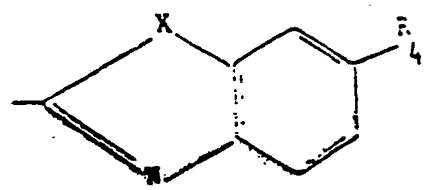

worin X Sauerstoff, eine Gruppe NH oder Schwefel sein kann und $R_4$ Wasserstoff oder ein Halogenatom, vorzugsweise Chlor, bezeichnet,

steht,

- $R_3$ Wasserstoff oder eine Hydroxylgruppe bezeichnet,
- n eine ganze Zahl zwischen 0 und 10 bedeutet, mit der Maßgabe, daß n ungleich Null ist, wenn $R_3$ = OH, und die Salze dieser Derivate, worin $R_1$ = H, mit Mineral- und organischen Basen zur Verwendung als entzündungshemmende Mittel.

3. Methylendiphosphonsäurederivate, dadurch gekennzeichnet, daß sie der folgenden Formel entsprechen:

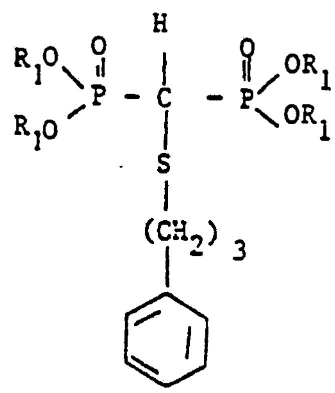

worin $R_1$ Wasserstoff oder eine Isopropylgruppe bedeutet.

4. Methylendiphosphonsäurederivate der Formel

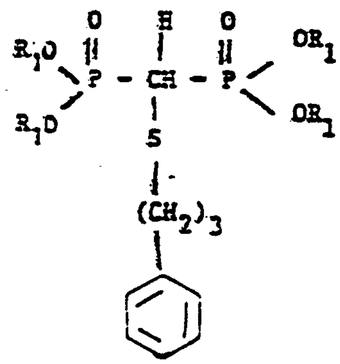

worin $R_1$ Wasserstoff oder eine Isopropylgruppe bedeutet, zur Verwendung als entzündungshemmende Mittel.

5. Methylendiphosphonsäurederivat, dadurch gekennzeichnet, daß es der folgenden Formel entspricht:

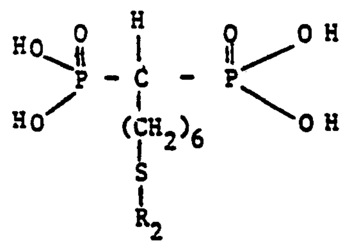

worin $R_2$ die Gruppe 1-Methylimidazol-2-yl ist.

6. Methylendiphosphonsäurederivat, dadurch gekennzeichnet, daß es der folgenden Formel entspricht:

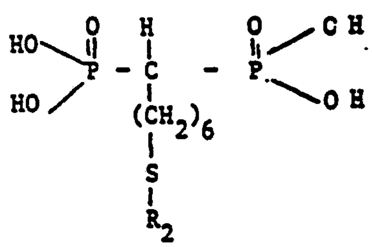

worin $R_2$ die Gruppe 1-Methylimidazol-2-yl ist, zur Verwendung als entzündungshemmendes Mittel.

7. Verfahren zur Herstellung der Produkte nach einem der Ansprüche 1 bis 6, worin $R_3$ Wasserstoff ist, dadurch gekennzeichnet, daß man als Ausgangsprodukt einen Niedrigalkylester der Methylendiphosphonsäure verwendet, daß man ihn durch Einwirken eines Natrisierungsmittels, wie Natriumhydrid, oder durch Einwirken von Kaliumkarbonat in Toluol in das entsprechende Metallderivat überführt, dann das Metallderivat mit einer Verbindung, ausgewählt aus den Produkten der Formel $R_2$-S-S-$R_2$, $R_2SCl$ und Hal-$(CH_2)_n$-S-$R_2$, worin Hal für ein Halogen steht, derart reagieren läßt, daß man zu einem Produkt der Formel (I) kommt, dann gegebenenfalls das Produkt der Formel (I), das in Esterform vorliegt, verseift und gegebenenfalls in eines seiner Salze überführt.

8. Verfahren nach Anspruch 7, dadurch gekennzeichnet, daß das natrisierte Derivat mit $R_2$-S-S-$R_2$ in einem Lösungsmittel, wie Toluol oder Dimethylformamid, bei einer Temperatur zwischen 20°C und der Kochtemperatur des Lösungsmittels zur Umsetzung gebracht wird.

9. Verfahren nach Anspruch 6, dadurch gekennzeichnet, daß das natrisierte Derivat mit Hal-$(CH_2)_n$-S-$R_2$ in einem Lösungsmittel, wie Toluol und Dimethylformamid, bei einer Temperatur zwischen 20°C und der Kochtemperatur des Lösungsmittels zur Umsetzung gebracht wird.

10. Verfahren zur Herstellung von Produkten nach Anspruch 1, worin n gleich 3 ist, dadurch gekennzeichnet, daß man als Ausgangsprodukt einen Niedrigalkylester der Methylendiphosphonsäure verwendet, den man mit einem Allylhalogenid reagieren läßt, daß man dann ein Thiol der Formel $SHR_2$ mit dem erhaltenen Produkt reagieren läßt und daß man das erhaltene Produkt gegebenenfalls verseift und in eines seiner Salze überführt.

11. Verfahren zur Herstellung der Produkte nach Anspruch 1, worin $R_3$ Hydroxyl ist, dadurch gekennzeichnet, daß es darin besteht, daß ein Säurechlorid der Formel $R_2S$-$(CH_2)_n$-COCl, worin $R_2$ die in Anspruch 1 angegebene Bedeutung hat, mit einem Trialkylphosphit bei einer Temperatur zwischen 20 und 50°C reagieren läßt, daß man das erhaltene Produkt mit einem Dialkylphosphit in Gegenwart eines sekundären Amins bei einer Temperatur zwischen 0 und 20°C behandelt, das erhaltene Produkt gegebenenfalls verseift und in eines seiner

Salze überführt.

12. Verfahren zur Herstellung der Produkte nach Anspruch 1, worin $R_3$ Hydroxyl ist, dadurch gekennzeichnet, daß es darin besteht, daß man eine Verbindung der Formel Hal-$(CH_2)_n$-COCl mit einem Trialkylphosphit bei einer Temperatur zwischen 20 und 50°C reagieren läßt, das erhaltene Produkt mit einem Dialkylphosphit in Gegenwart eines sekundären Amins bei einer Temperatur zwischen 0 und 20°C behandelt und die erhaltene Verbindung der Formel

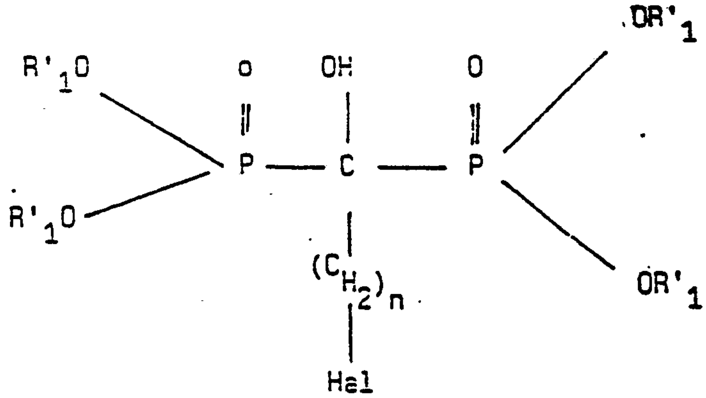

mit einem Thiol $R_2SH$ in einem Lösungsmittel, wie Acetonitril und in Gegenwart von 1,5-Diazabicyclo-(5,4,0)-5-undecen reagieren läßt, das erhaltene Produkt gegebenenfalls verseift und in eines seiner Salze überführt.

13. Medikamente zur Verwendung als entzündungshemmende Mittel, dadurch gekennzeichnet, daß sie ein Produkt nach einem der Ansprüche 2 oder 3 enthalten.

14. Medikamente nach Anspruch 10, dadurch gekennzeichnet, daß sie 10 bis 500 mg eines Produkts nach Anspruch 2 enthalten.

**Patentansprüche** für den Vertragsstaat AT:

1. Verfahren zur Herstellung von Methylendiphosphonsäurederivaten der Formel:

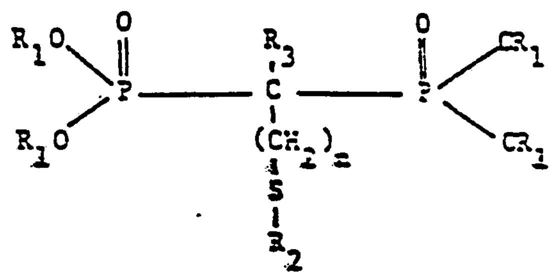

(I)

worin:

- $R_1$ Wasserstoff oder eine gerade oder verzweigte Niedrigalkylgruppe mit 1 bis 4 Kohlenstoffatomen darstellt;
- $R_2$ für
- Wasserstoff
- eine gegebenenfalls durch eine Hydroxyl-, Thiolgruppe, ein oder mehrere Halogenatome, eine Alkoxycarbonylgruppe oder eine Gruppe

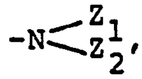

worin $Z_1$ und $Z_2$, einzeln betrachtet, Wasserstoff oder eine Niedrigalkylgruppe darstellen, substituierte Alkylgruppe;
- eine gegebenenfalls ein- oder mehrmals durch ein Halogen, eine Nitrogruppe, eine Niedrigalkyl-, Niedrigalkoxy-, Trifluormethylgruppe oder aber eine Gruppe $NH_2$ oder eine Gruppe COOH oder COO-Alkyl substituierte Phenylgruppe;
- eine Gruppe

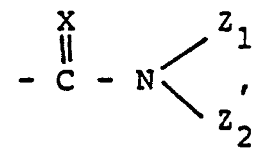

worin X Sauerstoff oder Schwefel bezeichnet und $Z_1$ und $Z_2$ obige Bedeutung haben;

- einen Heterocyclus mit 5 oder 6 Ketten, umfassend 1 oder 2 Heteroatome, ausgewählt aus Stickstoff oder Schwefel;
- einen Heterocyclus mit 5 Ketten, kondensiert mit einem Benzolkern und der Formel

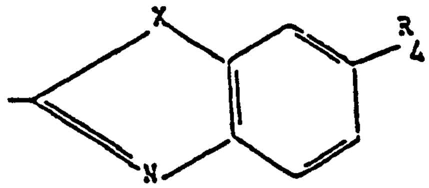

worin X Sauerstoff, eine Gruppe NH oder Schwefel sein kann und $R_4$ Wasserstoff oder ein Halogenatom, vorzugsweise Chlor,

bezeichnet,

steht,

- $R_3$ Wasserstoff bezeichnet,
- n eine ganze Zahl zwischen 0 und 10 bedeutet, mit der Maßgabe, daß
- $R_2$ keine Methyl- oder Pyridylgruppe ist, wenn $R_1$ = H und n = 0;
- $R_2$ keine Gruppe der Formel

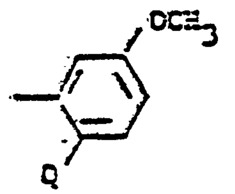

worin Q ein Halogenatom, eine Nitrogruppe oder eine Trifluormethylgruppe darstellt, ist, wenn $R_1$ eine Methyl- oder Äthylgruppe ist und n = 0;

- $R_2$ keine Phenylgruppe ist, wenn $R_1$ eine Äthylgruppe ist und n = 0;
- $R_2$ keine nichtsubstituierte Alkyl- oder Phenylgruppe oder Acetoxygruppe ist, wenn n = 0;

sowie der Salze dieser Derivate, worin $R_1$ = H, mit Mineral- und organischen Basen, dadurch gekennzeichnet,

daß man als Ausgangsprodukt einen Niedrigalkylester der Methylendiphosphonsäure verwendet, den man ihn durch Einwirken eines Natrisierungsmittels, wie Natriumhydrid, oder durch Einwirken von Kaliumkarbonat in Toluol in das entsprechende Metallderivat überführt, dann das Metallderivat mit einer Verbindung, ausgewählt aus den Produkten der Formel $R_2$-S-S-$R_2$, $R_2$SCl und Hal-$(CH_2)_n$-S-$R_2$, worin Hal für ein Halogen steht, derart reagieren läßt, daß man zu einem Produkt der Formel (I) kommt, dann gegebenenfalls das Produkt der Formel (I), das in Esterform vorliegt, verseift und gegebenenfalls in eines seiner Salze überführt.

2. Verfahren nach Anspruch 1, dadurch gekennzeichnet, daß das natrisierte Derivat mit $R_2$-S-S-$R_2$ in einem Lösungsmittel, wie Toluol oder Dimethylformamid, bei einer Temperatur zwischen 20°C und der Kochtemperatur des Lösungsmittels zur Umsetzung gebracht wird.

3. Verfahren nach Anspruch 1, dadurch gekennzeichnet, daß das natrisierte Derivat mit Hal-$(CH_2)_n$-S-$R_2$ in einem Lösungsmittel, wie Toluol und Dimethylformamid, bei einer Temperatur zwischen 20°C und der Kochtemperatur des Lösungsmittels zur Umsetzung gebracht wird.

4. Verfahren zur Herstellung von Methylendiphosphonsäurederivaten der Formel:

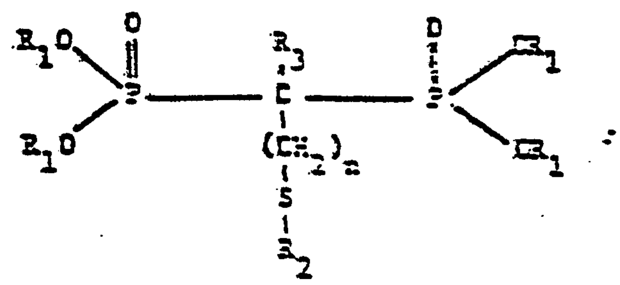

(I)

worin:

- $R_1$ Wasserstoff oder eine gerade oder verzweigte Niedrigalkylgruppe mit 1 bis 4 Kohlenstoffatomen

darstellt;

- $R_2$ für
- Wasserstoff
- eine gegebenenfalls durch eine Hydroxyl-, Thiolgruppe, ein oder mehrere Halogenatome, eine Alkoxycarbonylgruppe oder eine Gruppe

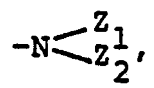

worin $Z_1$ und $Z_2$, einzeln betrachtet, Wasserstoff oder eine Niedrigalkylgruppe darstellen, substituierte Alkylgruppe;
- eine gegebenenfalls ein- oder mehrmals durch ein Halogen, eine Nitrogruppe, eine Niedrigalkyl-, Niedrigalkoxy-, Trifluormethylgruppe oder aber eine Gruppe $NH_2$ oder eine Gruppe COOH oder COO-Alkyl substituierte Phenylgruppe;
- eine Gruppe

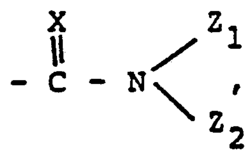

worin X Sauerstoff oder Schwefel bezeichnet und $Z_1$ und $Z_2$ obige Bedeutung haben;
- einen Heterocyclus mit 5 oder 6 Ketten, umfassend 1 oder 2 Heteroatome, ausgewählt aus Stickstoff oder Schwefel;
- einen Heterocyclus mit 5 Ketten, kondensiert mit einem Benzolkern und der Formel

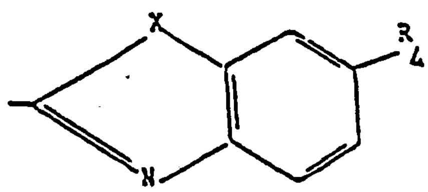

worin X Sauerstoff, eine Gruppe NH oder Schwefel sein kann und $R_4$ Wasserstoff oder ein Halogenatom, vorzugsweise Chlor, bezeichnet,

steht,
- $R_3$ Wasserstoff oder eine Hydroxylgruppe bezeichnet, dadurch gekennzeichnet, daß man als Ausgangsprodukt einen Niedrigalkylester der Methylendiphosphonsäure verwendet, den man mit einem Allylhalogenid reagieren läßt, daß man dann ein Thiol der Formel $R_2SH$ mit dem erhaltenen Produkt reagieren läßt und daß man das erhaltene Produkt gegebenenfalls verseift und in eines seiner Salze überführt.

5. Verfahren zur Herstellung von Methylendiphosphonsäurederivaten der Formel:

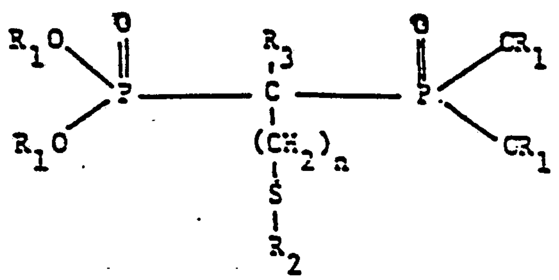

(I)

worin:
- $R_1$ Wasserstoff oder eine gerade oder verzweigte Niedrigalkylgruppe mit 1 bis 4 Kohlenstoffatomen darstellt;
- $R_2$ für
- Wasserstoff
- eine gegebenenfalls durch eine Hydroxyl-, Thiolgruppe, ein oder mehrere Halogenatome, eine Alkoxycarbonylgruppe oder eine Gruppe

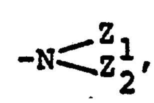

worin $Z_1$ und $Z_2$, einzeln betrachtet, Wasserstoff oder eine Niedrigalkylgruppe darstellen, substituierte Alkylgruppe;
- eine gegebenenfalls ein- oder mehrmals durch ein Halogen, eine Nitrogruppe, eine Niedrigalkyl-,

Niedrigalkoxy-, Trifluormethylgruppe oder aber eine Gruppe $NH_2$ oder eine Gruppe COOH oder COO-Alkyl substituierte Phenylgruppe;

- eine Gruppe

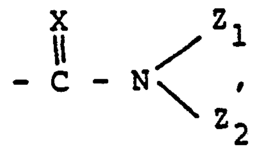

worin X Sauerstoff oder Schwefel bezeichnet und $Z_1$ und $Z_2$ obige Bedeutung haben;

- einen Heterocyclus mit 5 oder 6 Ketten, umfassend 1 oder 2 Heteroatome, ausgewählt aus Stickstoff oder Schwefel;

- einen Heterocyclus mit 5 Ketten, kondensiert mit einem Benzolkern und der Formel

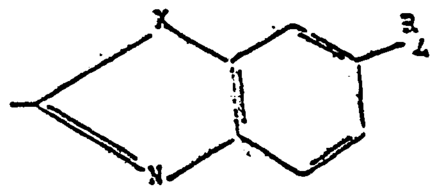

worin X Sauerstoff eine Gruppe NH oder Schwefel sein kann und $R_4$ Wasserstoff oder ein Halogenatom, vorzugsweise Chlor, bezeichnet,

steht,

- $R_3$ eine Hydroxylgruppe ist,
- n eine ganze Zahl zwischen 1 und 0 darstellt, mit der Maßgabe, daß:
- $R_2$ keine Methylgruppe ist, wenn $n = 1$;

sowie der Salze dieser Derivate, worin $R_1 = H$, mit Mineralund organischen Basen, dadurch gekennzeichnet, daß es darin besteht, daß ein Säurechlorid der Formel $R_2S\text{-}(CH_2)_n\text{-}COCl$, worin $R_2$ die in Anspruch 1 angegebene Bedeutung hat, mit einem Trialkylphosphit bei einer Temperatur zwischen 20 und 50°C reagieren läßt, daß man das erhaltene Produkt mit einem Dialkylphosphit in Gegenwart eines sekundären Amins bei einer Temperatur zwischen 0 und 20°C behandelt, das erhaltene Produkt gegebenenfalls verseift und in eines seiner Salze überführt.

6. Verfahren zur Herstellung der Methylendiphosphonsäurederivate gemäß Anspruch 5, dadurch gekennzeichnet, daß es darin besteht, daß man eine Verbindung der Formel $Hal\text{-}(CH_2)_n\text{-}COCl$ mit einem Trialkylphosphit bei einer Temperatur zwischen 20 und 50°C reagieren läßt, das erhaltene Produkt mit einem Dialkylphosphit in Gegenwart eines sekundären Amins bei einer Temperatur zwischen 0 und 20°C behandelt und die erhaltene Verbindung der Formel

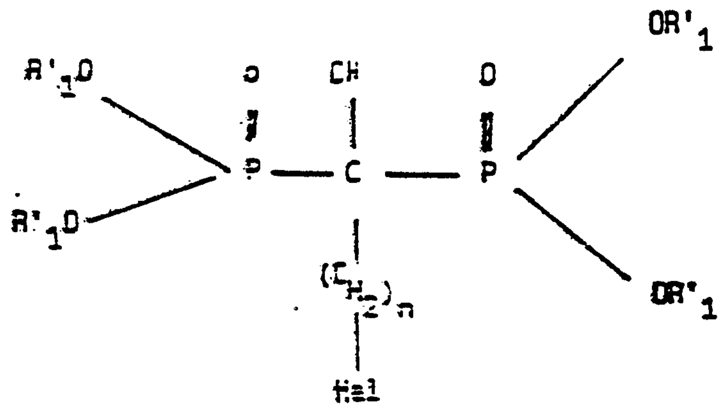

mit einem Thiol $R_2SH$ in einem Lösungsmittel, wie Acetonitril und in Gegenwart von 1,5-Diazabicyclo-(5,4,0)-5-undecen reagieren läßt, das erhaltene Produkt gegebenenfalls verseift und in eines seiner Salze überführt.

7. Verfahren zur Herstellung von Methylendiphosphonsäurederivaten der Formel

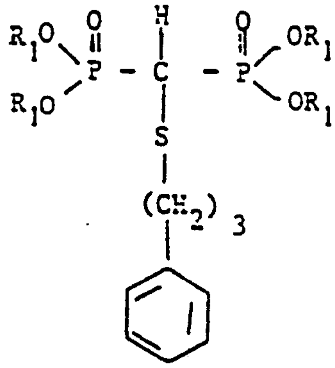

worin $R_1$ Wasserstoff oder eine Isopropylgruppe ist, dadurch gekennzeichnet, daß man als Ausgangsprodukt Tetraisopropylmethylendiphosphonat einsetzt, welches man durch Einwirken von Kaliumkarbonat in Toluol in das entsprechende Metallderivat überführt, dann das Metallderivat mit Di-(3-phenylpropyl)-disulfid reagieren läßt und dann gegebenenfalls die erhaltene Verbindung verseift.

8. Verfahren zur Herstellung von Methylendiphosphonsäurederivaten der Formel

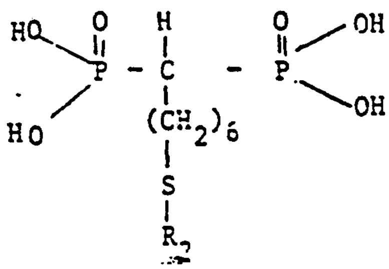

worin $R_2$ die Gruppe 1-methylimidazol-2-yl ist dadurch gekennzeichnet, daß man als Ausgangsprodukt Tetraisopropylmethylendiphosphat einsetzt, welches man durch Einwirken von Natriumhydrid in das entsprechende Metallderivat überführt, dann das Metallderivat mit 1,6-Dibromhexan reagieren läßt, das erhaltene bromierte Derivat danach mit 2-Mercapto-1-methylimidazol zur Umsetzung bringt.

9. Verwendung der Methylendiphosphonsäurederivate der Formel

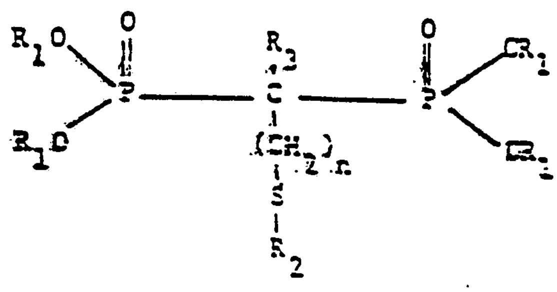

(I)

worin:

- $R_1$ Wasserstoff oder eine gerade oder verzweigte Niedrigalkylgruppe mit 1 bis 4 Kohlenstoffatomen darstellt;

- $R_2$ für

- Wasserstoff

- eine gegebenenfalis durch eine Hydroxyl-, Thiolgruppe, ein oder mehrere Halogenatome, eine Alkoxycarbonylgruppe oder eine Gruppe

$$-N<^{Z_1}_{Z_2},$$

worin $Z_1$ und $Z_2$, einzeln betrachtet, Wasserstoff oder eine Niedrigalkylgruppe darstellen, substituierte Alkylgruppe;

- eine gegebenenfalls ein- oder mehrmals durch ein Halogen, eine Nitrogruppe, eine Nirdrigalkyl-, Niedrigalkoxy-, Trifluormethylgruppe oder aber eine Gruppe $NH_2$ oder eine Gruppe COOH oder COO-Alkyl substituierte Phenylgruppe;

- eine Gruppe

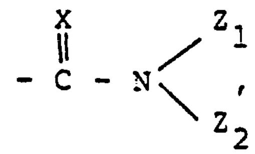

worin X Sauerstoff oder Schwefel bezeichnet und $Z_1$ und $Z_2$ obige Bedeutung haben;

- einen Heterocyclus mit 5 oder 6 Ketten, umfassend 1 oder 2 Heteroatome, ausgewählt aus Stickstoff oder Schwefel;
- einen Heterocyclus mit 5 Ketten, kondensiert mit einem Benzolkern und der Formel

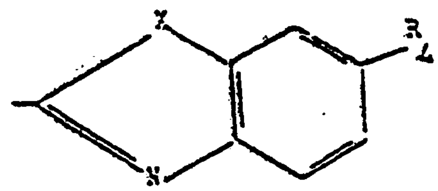

worin X Sauerstoff, eine Gruppe NH oder Schwefel sein kann und $R_4$ Wasserstoff oder ein Halogenatom, vorzugsweise Chlor bezeichnet,

steht,

- $R_3$ Wasserstoff oder eine Hydroxylgruppe bezeichnet,
- n eine ganze Zahl zwischen 0 und 10 bedeutet, mit der Maßgabe, daß n ungleich Null ist, wenn $R_3$ = OH, und der Salze dieser Derivate, worin $R_1$ = H, mit Mineral- oder organischen Basen, zur Herstellung von Medikamenten, die als entzündungshemmende Mittel nützlich sind.

10. Verwendung der Methylendiphosphonsäurederivate der Formel

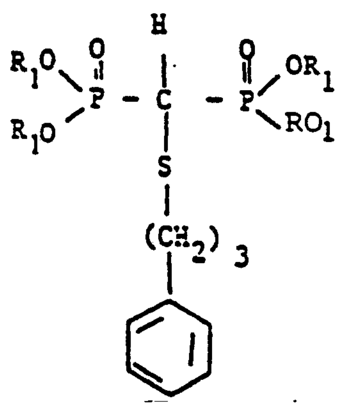

worin $R_1$ Wasserstoff oder eine Isopropylgruppe bedeutet, oder der Formel

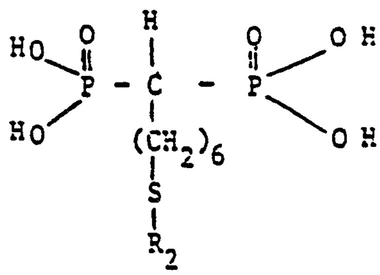

worin $R_2$ die Gruppe 1-Methylimidazol-2-yl ist, zur Herstellung von Medikamenten die als entzündungshemmende Mittel nützlich sind.

**Claims** for the Contracting States: BE, CH, DE, FR, GB, IT, LI, LU, NL, SE.

1. Derivatives of methylene-diphosphonic acid of the formula

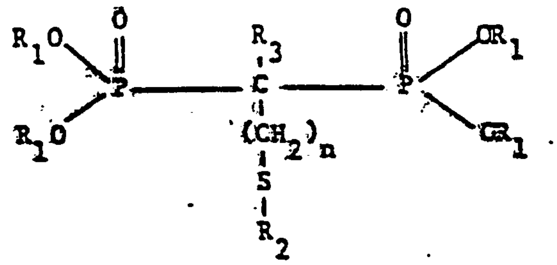

(I)

in which:

- $R_1$ is hydrogen or a straight or branched lower alkyl group having from 1 to 4 carbon atoms,
- $R_2$ is:

. hydrogen,

. an alkyl group which is possibly substituted by a hydroxyl group, a thiol group, one or more halogen atoms, an alkoxycarbonyl group or a

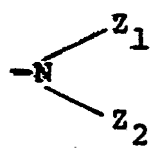

group, where $Z_1$ and $Z_2$, considered independently of one another, are hydrogen or a lower alkyl group,

. a phenyl group which is possibly substituted one or more times by halogen, a nitro group, a lower alkyl group, a lower alkoxy group, trifluoromethyl, an $NH_2$ group, a COOH group or a COO alkyl group,

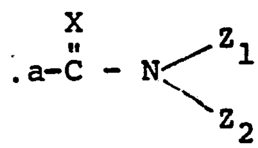

group, where X is oxygen or sulfur and $Z_1$ and $Z_2$ are as defined above,

. a heterocyclic radical with 5 or 6 members, containing 1 or 2 heteroatoms chosen from amongst nitrogen and sulfur, or

. a heterocyclic radical with 5 members fused to a benzene ring and having the formula

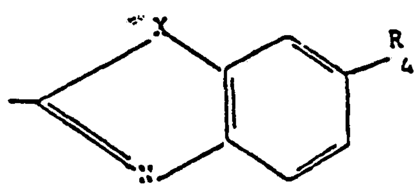

where X is oxygen, an NH group or sulfur and $R_4$ is hydrogen or a halogen atom, preferably chlorine,

- $R_3$ is hydrogen or a hydroxyl group, and
- n is an integer between 0 and 10, with the proviso that:

n cannot be 0 if $R_3$ is OH

$R_2$ is different from the methyl or pyridyl groups when $R_1 = R_3 = H$ and $n = 0$;

$R_2$ is different from the group of formula:

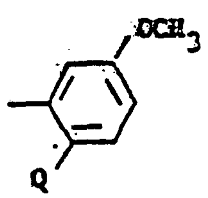

in which Q is a halogen atom, a nitro group or a trifluoromethyl group when $R_1$ is the methyl or ethyl group, $R_3 = H$ and $n = 0$,

$R_2$ is different from the phenyl group when $R_1$ is the ethyl group, $R_3 = H$ and $n = 0$;

$R_2$ is not an unsubstituted alkyl or phenyl group or an acetoxy group when $R_3$ is H and n = 1,
$R_2$ is not the methyl group when $R_3$ = OH and n = 1; as well as the salts of said derivates in which $R_1$ = H with mineral and organic bases.

2. Derivatives of methylene-diphosphonic acid of formula

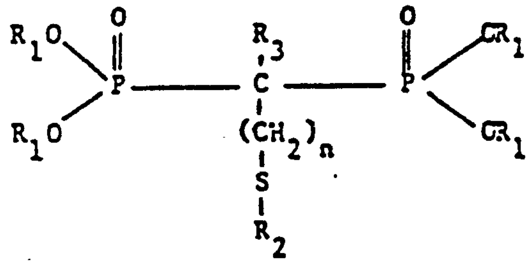

(I)

in which:

-$R_1$ represents hydrogen or a straight or branched lower alkyl group having from 1 to 4 atoms of carbon;

-$R_2$ represents:

. hydrogen

. an alkyl group which is possibly substituted by a hydroxyl group, a thiol group, one or more halogen atoms, an alkoxycarbonyl group or a

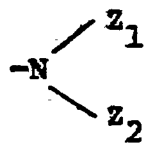

group, where $Z_1$ and $Z_2$, considered independently of one another, are hydrogen or a lower alkyl group,

. a phenyl group which is possibly substituted one or more times by halogen, a nitro group, a lower alkyl group, a lower alkoxy group, trifluoromethyl, an $NH_2$ group, a COOH group or a COO alkyl group,

. a 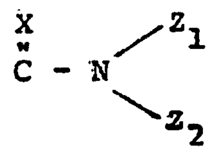

group, where X is oxygen or sulfur and $Z_1$ and $Z_2$ are as defined above,

. a heterocyclic radical with 5 or 6 members, containing 1 or 2 heteroatoms chosen from amongst nitrogen and sulfur, or

. a heterocyclic radical with 5 members fused to a benzene ring and having the formula

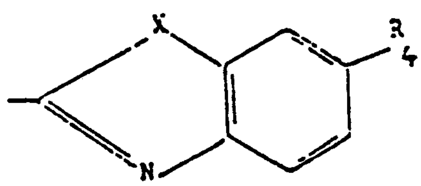

where X is oxygen, an NH group or sulfur and $R_4$ is hydrogen or a halogen atom, preferably chlorine,

- $R_3$ is hydrogen or a hydroxyl group, and

- n is an integer between 0 and 10, with the proviso that n cannot be 0 if $R_3$ is OH and the salts of said derivatives in which $R_1$ = H with mineral or organic acids for their use as anti-inflammatory agents.

3. Derivatives of methylene-diphosphonic acid characterised in that they respond to the formula:

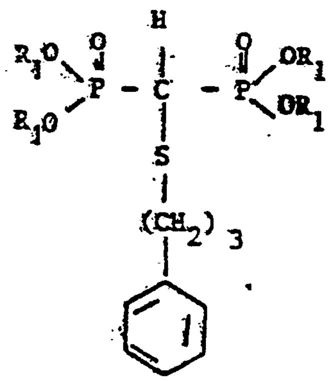

in which $R_1$ is hydrogen or the isopropyl group.

4. Derivatives of methylene-diphosphonic acid of formula

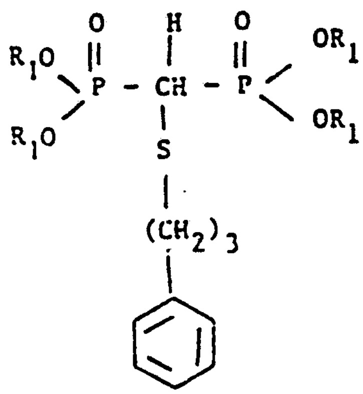

in which $R_1$ is hydrogen or the isopropyl group for their use as anti-inflammatory agents.

5. Derivative of methylene-diphosphonic acid characterized in that it responds to the following formula:

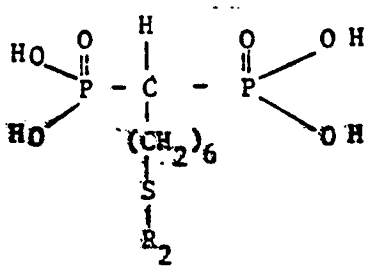

in which $R_2$ is the 1-methyl imidazol-2-yl group.

6. Derivative of methylene-diphosphonic acid characterized in that it responds to the formula:

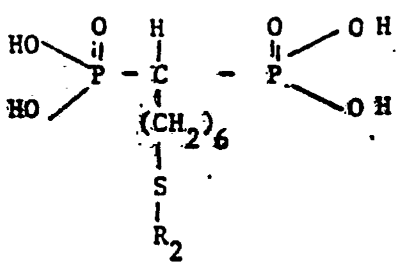

in which $R_2$ is the 1-methyl imidazol-2-yl group for its use as anti-inflammatory agent.

7. Process for the preparation of products according to any one of claims 1 to 6 in which $R_3$ is hydrogen, characterized in that there is used, as the starting material, a lower alkyl ester of methylene-diphosphonic acid,

which is converted to the corresponding metallic derivative by reaction with a sodium-introducing agent such as sodium hydride, or by reaction with potash in the toluene, after which the said metallic-containing product is reacted with a compound chosen from amongst the products of the formula $R_2$-S-S-$R_2$, $R_2SCl$ and Hal-$(CH_2)_n$-S-$R_2$, where Hal is a halogen, so as to give a product of the formula (I), then said product of the formula (I) which is in the form of an ester is optionally saponified and optionally converted into one of its salts.

8. Process according to claim 7, characterized in that the sodium containing derivative is reacted with $R_2$-S-S-$R_2$ in a solvent such as toluene or dimethylformamide at a temperature comprised between 20°C and the boiling temperature of the solvent.

9. Process according to claim 6, characterized in that the sodium-containing derivative is reacted with Hal-$(CH_2)_n$-S-$R_2$ in a solvent such as toluene and dimethylformamide at a temperature comprised between 20°C and the boiling temperature of the solvent.

10. Process for the preparation of products according to claim 1, in which n is equal to 3, characterized in that there is used, as the starting material, a lower alkyl ester of the methylene-diphosphonic acid which is reacted on an allyl halide, after which the product obtained is reacted with a thiol of the formula $SHR_2$, and in that the product obtained is optionally saponified and converted into one of its salts.

11. Process for obtaining products according to claim 1, in which $R_3$ is hydroxyl, characterized in that it consists in reacting an acid chloride of formula $R_2S$-$(CH_2)_n$-COCl in which $R_2$ is such as defined in claim 1, with a trialkylphosphite at a temperature comprised between 20 and 50°, in treating the product thus obtained by a dialkylphosphite in the presence of a secondary amine at a temperature comprised between 0 and 20°C, optionally in saponifying the resulting product and in converting it into one of its salts.

12. Process for obtaining products according to claim 1, in which $R_3$ is hydroxyl, characterized in that it consists in reacting a compound of formula: Hal-$(CH_2)_n$-COCl with a trialkylphosphite at a temperature comprised between 20 and 50°C, in treating the product obtained with a dialkylphosphite in the presence of a secondary amine at a temperature comprised between 0 and 20°C, and in reacting the resulting compound of formula:

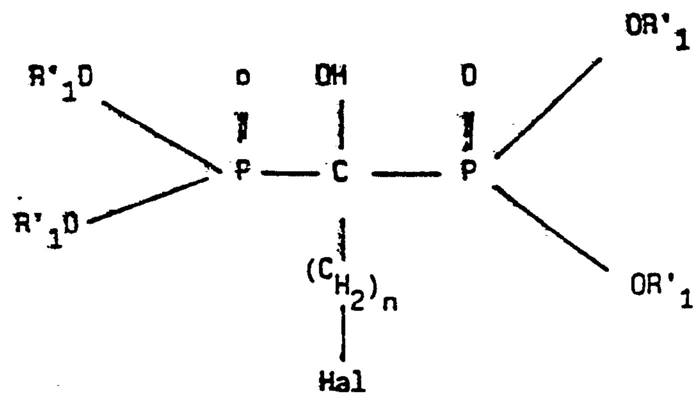

with a $R_2SH$ thiol in a solvent such as acetonitrile and in the presence of 1,5-diaza-bicyclo-(5,4,0)-undec-5-ene, optionally in saponifying the resulting product and converting it into one of its salts.

13. Drugs for the use as anti-inflammatory agents characterized in that they contain a product according to any one of claims 2 or 3.

14. Drugs according to claim 10, characterized in that they contain from 10 to 500 mg of a product according to claim 2.

**Claims** for the contracting state: AT

1. Process for the preparation of derivatives of methylene-diphosphonic acid of the formula:

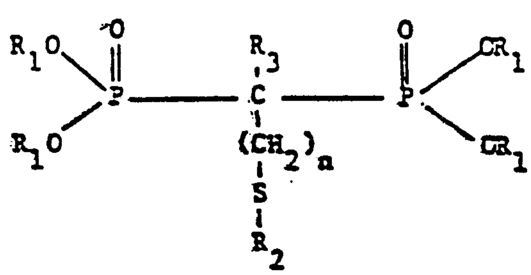

(I)

in which:
- $R_1$ is hydrogen or a straight or branched lower alkyl group having from 1 to 4 carbon atoms,
- $R_2$ is:
- hydrogen
- an alkyl group which is possibly substituted by a hydroxyl group, a thiol group, one or more halogen atoms, an alkoxycarbonyl group or

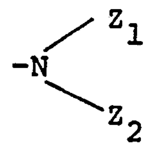

group, where $Z_1$ and $Z_2$, considered independently of one another, are hydrogen or a lower alkyl group,
- a phenyl group which is possibly substituted one or more times by halogen, a nitro group, a lower alkyl group, a lower alkoxy group, trifluoromethyl, an $NH_2$ group, a COOH group or a COO alkyl group,

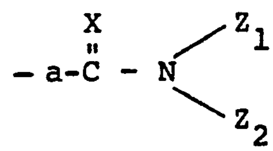

group, where X is oxygen or sulfur and $Z_1$ and $Z_2$ are as defined above,
- a heterocyclic radical with 5 or 6 members, containing 1 or 2 heteroatoms chosen from amongst nitrogen and sulfur,
- a heterocyclic radical with 5 members fused to a benzene ring and having the formula

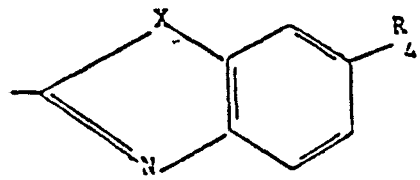

where X is oxygen an NH group or sulfur and $R_4$ is hydrogen or a halogen atom, preferably chlorine,
- $R_3$ is hydrogen,
- n is an integer between 0 and 10, with the proviso that:
- $R_2$ is different from the methyl or pyridyl groups when $R_1$ = H and n = 0;
- $R_2$ is different from the group of formula:

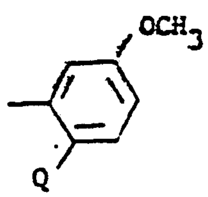

in which Q is a halogen atom, a nitro group or a trifluoromethyl group when $R_1$ is the methyl or ethyl group, and n = 0,
- $R_2$ is different from the phenyl group when $R_1$ is the ethyl group, and n = 0;
- $R_2$ is not an unsubstituted alkyl or phenyl group or an acetoxy group when n = 1,
as well as the salts of said derivatives in which $R_1$ = H with mineral and organic bases, characterized in that there is used, as the starting material, a lower alkyl ester of methylene-diphosponic acid, which is converted to the corresponding metallic derivative by reaction with a sodium-introducing agent such as sodium hydride, or by reaction with potash in the toluene, after which the said metallic-containing product is reacted with a compound chosen from amongst the products of the formula $R_2$-S-S-$R_2$, $R_2$SCl and Hal-$(CH_2)_n$-S-$R_2$, where Hal is a halogen, so as to give a product of the formula (I), then said product of the formula (I) which is in the form of an ester is optionally saponified and optionally converted into one of its salts.

2. A process according to claim 1, characterized in that the sodium-containing product is reacted with $R_2$-S-S-$R_2$ in a solvent such as toluene or dimethylformamide at a temperature of between 20°C and the boiling temperature of the solvent.

3. A process according to claim 1, characterized in the sodium-containing product is reacted with Hal-$(CH_2)_n$-S-$R_2$ in a solvent such as toluene or dimethylformamide at a temperature of between 20°C and the boiling temperature of the solvent.

4. Process for the preparation of derivatives of methylene-diphosphonic acid of formula

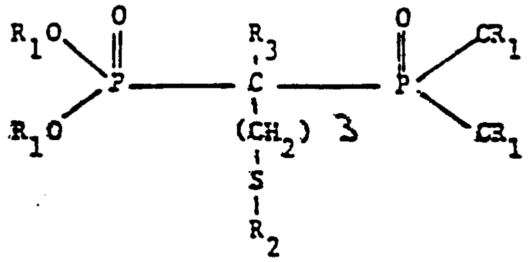

(I)

in which:
- $R_1$ represents hydrogen or a straight or branched lower alkyl group having from 1 to 4 atoms of carbon;
- $R_2$ represents:
- hydrogen;
- an alkyl group which is possibly substituted by a hydroxyl group, a thiol group, one or more halogen atoms, an alcoxycarbonyl group or a

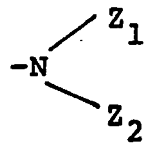

group, where $Z_1$ and $Z_2$, considered independently of one another, are hydrogen or a lower alkyl group,
- a phenyl group which is possibly substituted one or more times by halogen, a nitro group, a lower alkyl group, a lower alkoxy group, trifluoromethyl, an $NH_2$ group, a COOH group or a COO alkyl group,

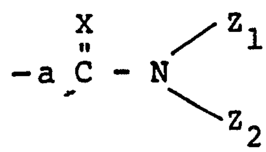

group, where X is oxygen or sulfur and $Z_1$ and $Z_2$ are as defined above,
- a heterocyclic radical with 5 or 6 members, containing 1 or 2 heteroatoms chosen from amongst nitrogen and sulfur, or
- a heterocyclic radical with 5 members fused to a benzene ring and having the formula

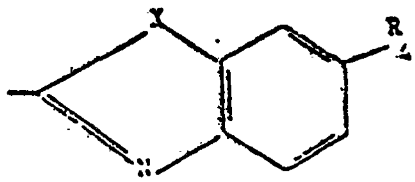

where X is oxygen an NH group or sulfur and $R_4$ is hydrogen or a halogen atom, preferably chlorine, $R_3$ is hydrogen or a hydroxyl group, characterized in that there is used as starting product a lower alkyl ester of methylene-diphosphonic acid, which is reacted with an allyl halide, after which the product obtained is reacted with a thiol of formula $R_2SH$, and in that the resulting product is optionally saponified and converted into one of its salts.

5. Process for the preparation of derivatives of methylene-diphosphonic acid of formula:

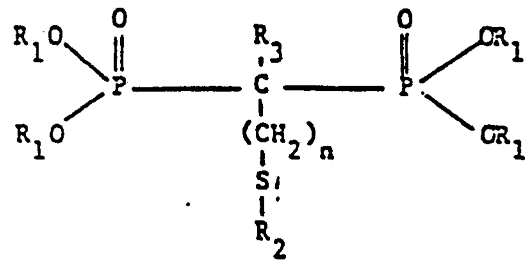

(I)

in which:

- $R_1$ is hydrogen or a straight or branched lower alkyl group having from 1 to 4 carbon atoms,
- $R_2$ is:
- hydrogen,
- an alkyl group which is possibly substituted by a hydroxyl group, a thiol group, one or more halogen atoms, an alkoxycarbonyl group or a

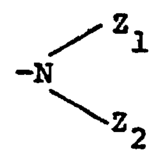

group, where $Z_1$ and $Z_2$, considered independently of one another, are hydrogen or a lower alkyl group,
- a phenyl group which is possibly substituted one or more times by halogen, a nitro group, a lower alkyl group, a lower alkoxy group, trifluoromethyl, an $NH_2$ group, a COOH group or a COO alkyl group,

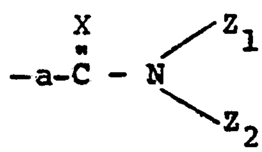

group, where X is oxygen or sulfur and $Z_1$ and $Z_2$ are as defined above,
- a heterocyclic radical with 5 or 6 members, containing 1 or 2 heteroatoms chosen from amongst nitrogen and sulfur, or
- a heterocyclic radical with 5 members fused to a benzene ring and having the formula

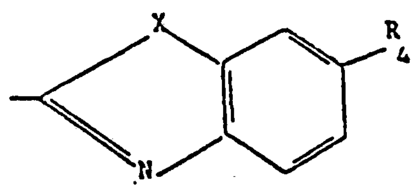

where X is oxygen, an NH group or sulfur and $R_4$ is hydrogen or a halogen atom, preferably chlorine,
- $R_3$ is a hydroxyl group, and
- n is an integer between 1 and 10, with the proviso that:
- $R_2$ is not the methyl group when n = 1;

as well as the salts of said derivatives in which $R_1 = H$ with mineral and organic bases,

characterized in that it consists in reacting an acid chloride of formula $R_2S\text{-}(CH_2)_n\text{-}COCl$ in which $R_2$ is such as defined in claim 1 with a trialkylphosphite at a temperature comprised between 20°C and 50°C, then in treating the product obtained with a dialkylphosphite in the presence of a secondary amine at a temperature comprised between 0 and 20°C, optionally in saponifying the product obtained and in converting it into one of its salts.

6. Process for the preparation of derivatives of methylene-diphosphonic acid such as defined in claim 5, characterized in that it consists in reacting a compound of formula: $Hal\text{-}(CH_2)_n\text{-}COCl$ with a trialkylphosphite at a temperature comprised between 20°C and 50°C, in that the product obtained is treated with a dialkylphosphite in the presence of a secondary amine at a temperature comprised between 0 and 20°C, and in reacting the compound obtained of formula:

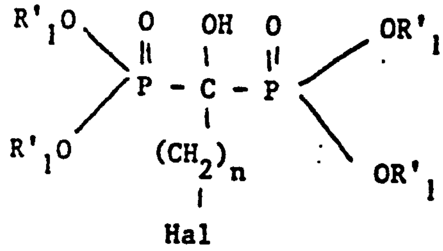

with a thiol of formula $R_2SH$ in a solvent such as acetonitrile and in the presence of 1,5-diaza-bicyclo (5,4,0)-undec-5-ene, optionally in saponifying the product obtained and converting it into one of its salts.

7. Process for the preparation of derivatives of methylene-diphosphonic acid of formula

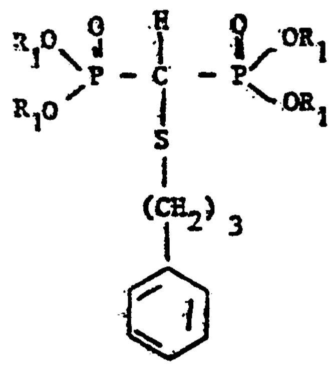

in which $R_1$ is hydrogen or the isopropyl group, characterized in that there is used as starting product the tetraisopropyl methylene-diphosphonate which is converted into the corresponding metallic derivative by reaction with potash in the toluene, then said metallic derivative is reacted with the di(3-phenyl propyl) disulphide, then the resulting compound is optionally saponified.

8. Process for the preparation of derivatives of methylene-diphosphonic acid of formula:

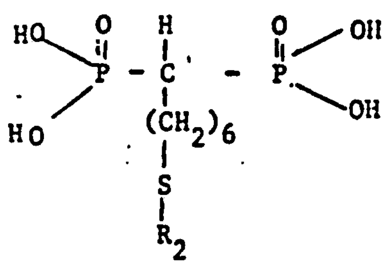

in which $R_2$ is the 1-methyl imidazol-2-yl group characterized in that there is used as starting product the tetraisopropyl methylenediphosphate which is converted into the corresponding metallic derivative by reaction with the sodium hydride, then the metallic derivative is reacted with the 1,6-dibromo hexane, the bromine-containing derivative is then reacted with the 2-mercapto 1-methyl imidazole.

9. Use of derivatives of methylene-diphosphonic acid of formula:

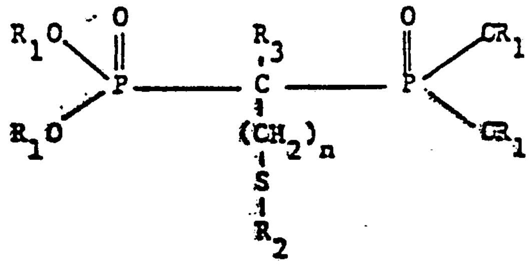

(I)

in which:

- $R_1$ represents hydrogen of a straight or branched lower alkyl group having from 1 to 4 carbon atoms;
- $R_2$ represents:
- hydrogen
- an alkyl group which is possibly substituted by a hydroxyl group, thiol, one or more halogen atoms, an alkoxycarbonyl group or a

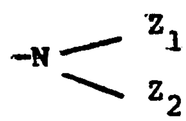

group, where $Z_1$ and $Z_2$ considered independently of one another represent hydrogen or a lower alkyl group;
- a phenyl group which is possibly substituted one or more times by halogen, a nitro group, a lower alkyl group, a lower alkoxy group, trifluoromethyl or a $NH_2$ group, or a COOH or a COO alkyl group;

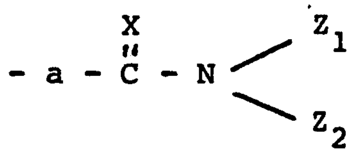

group, where X is oxygen or sulfur, and $Z_1$ and $Z_2$ are as defined above;
- a heterocyclic radical with 5 or 6 members, containing 1 or 2 heteroatoms chosen from amongst nitrogen and sulfur;
- a heterocyclic radical with 5 members fused to a benzene ring and having the formula:

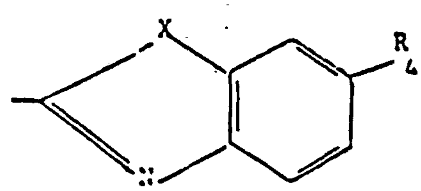

where X is oxygen, an NH group or sulfur and $R_4$ designates hydrogen or a halogen atom, preferably chlorine,
- $R_3$ designates hydrogen or a hydroxyl group,
- n represents an integer between 0 and 10 with the proviso that n cannot be 0 if $R_3$ = OH and the salts of said derivatives in which $R_1$ = H with mineral or organic bases for the preparation of drugs useful as anti-inflammatory agents.

10. Use of the derivatives of methylene-diphosphonic acid of formula:

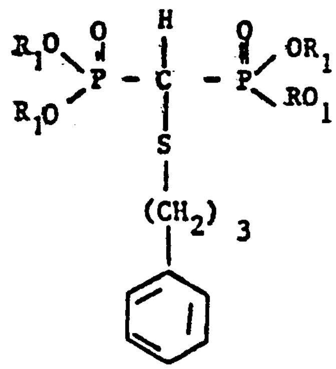

in which $R_1$ is hydrogen or the isopropyl group, or of formula:

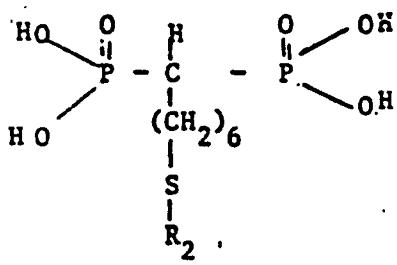

in which $R_2$ is the 1-methyl imidazol-2-yl group for the preparation of drugs useful as anti-inflammatory agents.